# EUROPEAN PATENT APPLICATION

(11) **EP 4 105 319 A1**
(43) Date of publication of application: **21.12.2022**
(21) Application number: 21754008.7
(22) Date of filing: 12.02.2021
(51) Int. Cl.: C12N 5/0735, C12N 5/10, C12N 9/99

(54) **METHOD FOR SUPPRESSING DIFFERENTIATION OF PLURIPOTENT STEM CELLS**

(30) Priority: 12.02.2020 JP 2020021843
(71) Applicant: Kaneka Corporation, Osaka-shi, Osaka 530-8288 (JP); RIKEN, Wako-shi, Saitama 351-0198 (JP)
(72) Inventor: TAKEUCHI, Kazuhiro, Kobe-shi, Hyogo 650-0047 (JP); IBUKI, Masato, Kobe-shi, Hyogo 650-0047 (JP); HAYASHI, Yohei, Wako-shi, Saitama 351-0198 (JP); TAKASAKI, Mami, Wako-shi, Saitama 351-0198 (JP); KAMBAYASHI, Sho, Kobe-shi, Hyogo 650-0047 (JP); KAWAI, Yoshikazu, Kobe-shi, Hyogo 650-0047 (JP)
(74) Representative: Hoffmann Eitle
(86) International application number: PCT/JP2021/005255
(87) International publication number: WO 2021/162090

(57) **Abstract**

Pluripotent stem cells are suspension-cultured with the undifferentiated state thereof maintained. In suspension culture of pluripotent stem cells, the undifferentiated state is maintained by the presence of a PKC inhibitor, especially, a PKCβ inhibitor, and a tankyrase inhibitor (TNKS inhibitor).

## Description

### Technical Field

The present invention relates to a production method of producing pluripotent stem cells by suspension culture, and to a production method of producing a pluripotent stem cell population by suspension culture.

### Background Art

Pluripotent stem cells such as ES cells and iPS cells have an ability to proliferate indefinitely and an ability to differentiate into various somatic cells. Putting therapeutic methods to transplant somatic cells obtained by inducing differentiation of pluripotent stem cells into practical use possibly leads to fundamental revolution for therapeutic methods for refractory diseases and lifestyle diseases. For example, techniques to induce pluripotent stem cells *in vitro* to differentiate into nerve cells and a wide variety of somatic cells including cardiomyocytes, blood cells, and retinal cells have been already developed.

On the other hand, regenerative medicine using pluripotent stem cells still have problems for putting it into practical use, and one of the problems lies in productivity for pluripotent stem cells. For example, it is said that approximately 2 × 10¹¹ cells are required for regeneration of livers. Methods of culturing pluripotent stem cells are roughly classified into adherent culture, in which cells are cultured with the cells adhered on a flat substrate, and suspension culture, in which cells are cultured with the cells suspended in liquid medium. To culture the mentioned number of cells by adherent culture, a substrate of 10⁶ cm² or larger is needed, which corresponds to approximately 20,000 common 10-cm dishes. The number of cells to be obtained by adherent culture on the surface of a substrate depends on the culture area, and hence a huge area is required for scall-up, thus it is difficult to supply cells in an amount required for regenerative medicine. The number of cells to be obtained by suspension culture depends on the volume of medium because cells are cultured with the cells suspended in liquid medium. Accordingly, suspension culture allows easy scale-up, thus being suitable for mass production of cells. For example, Non Patent Literature 1 discloses a method of suspension-culturing pluripotent stem cells while liquid medium is stirred with use of a spinner flask as a cell culture vessel for suspension culture.

Another problem for putting the regenerative medicine into practical use is productivity for target somatic cells. Examples of attempts to efficiently produce target somatic cells include an attempt to enhance the efficiency of induction of differentiation, and various methods therefor have been reported. For example, Non Patent Literature 2 discloses a method of suppressing the spontaneous differentiation of pluripotent stem cells in adherent culture of pluripotent stem cells by adding a protein kinase C (PKC) inhibitor into medium. Non Patent Literature 3 discloses a method of suppressing the spontaneous differentiation of pluripotent stem cells in adherent culture of pluripotent stem cells by adding a tankyrase (TNKS) inhibitor into medium.

As described so far, various methods for efficiently producing target somatic cells from pluripotent stem cells have been developed. However, the methods of Non Patent Literatures 2 and 3 are described only for adherent culture, the suppressive effect on spontaneous differentiation in suspension culture is not described, and the effect of combining a PKC inhibitor and a TNKS inhibitor is neither described nor suggested.

### Citation List

### Non Patent Literature

Non Patent Literature 1: Olmer R. et al., Tissue Engineering: Part C, Volume 18(10):772-784(2012)
Non Patent Literature 2: M. Kinehara et al., PLoS One. 2013; 8(1):e54122
Non Patent Literature 3: T. Sumi et al., PLoS One. 2013; 8(5):e63378

### Summary of Invention

### Technical Problem

An object of the present invention is to provide a technique to suppress the spontaneous differentiation into any of the three germ layers, endoderm, mesoderm, and ectoderm, and maintain the undifferentiated state of pluripotent stem cells.

### Solution to Problem

The present inventors diligently examined to achieve the object, and succeeded in suppressing the spontaneous differentiation into any of the three germ layers, and maintaining the undifferentiated state of pluripotent stem cells in suspension culture of pluripotent stem cells by the coexistence of a PKCβ inhibitor and a TNKS inhibitor. The present invention was completed on the basis of those research results, and includes the followings.
(1) A method for producing a pluripotent stem cell population, the method comprising the step of performing suspension culture of a pluripotent stem cell in a liquid medium comprising a PKCβ inhibitor and a TNKS inhibitor.
(2) The method according to (1), wherein the concentration of the PKCβ inhibitor comprised in the liquid medium is 25 nM or more and 15 µM or less.
(3) The method according to (1), wherein the concentration of the TNKS inhibitor comprised in the liquid medium is 90 nM or more and 40 µM or less.
(4) The method according to (1), wherein the ratio of the concentrations of the PKCβ inhibitor and the TNKS inhibitor comprised in the liquid medium is in the range of 167:1 or higher and 1:1600 or lower.
(5) The method according to (1), wherein the liquid medium comprises at least one selected from the group consisting of L-ascorbic acid, insulin, transferrin, selenium, and sodium hydrogen carbonate.
(6) The method according to (1), wherein the liquid medium comprises FGF2 and/or TGF-β1.
(7) The method according to (1), wherein the liquid medium comprises a ROCK inhibitor.
(8) The method according to (7), wherein the ROCK inhibitor is Y-27632.
(9) The method according to (1), wherein the step of performing suspension culture comprises the step of forming a cell aggregate.
(10) The method according to (1), wherein the step of performing suspension culture comprises the step of collecting a cell aggregate.
(11) The method according to (1), wherein, in the pluripotent stem cell population, the proportion of cells positive for OCT4 is 90% or higher, the proportion of cells positive for SOX2 is 90% or higher, and the proportion of cells positive for Nanog is 90% or higher.
(12) The method according to (1), wherein the pluripotent stem cell is an ES cell and/or an induced pluripotent stem cell.
(13) The method according to (1), wherein the suspension culture is performed until lactic acid concentration in the liquid medium reaches 5 to 15 mM.
(14) The method according to (1), wherein the suspension culture is stirring culture with a blade tip speed of 0.05 m/s or higher and 1.37 m/s or lower.
(15) A pluripotent stem cell population produced by the method according to any of (1) to (14).
(16) A differentiation inhibitor for pluripotent stem cells, the differentiation inhibitor comprising a PKCβ inhibitor and a TNKS inhibitor.
(17) The differentiation inhibitor for pluripotent stem cells according to (16), wherein the concentration of the PKCβ inhibitor comprised therein is 50 nM or more and 200 mM or less.
(18) The differentiation inhibitor for pluripotent stem cells according to (16), wherein the concentration of the TNKS inhibitor comprised therein is 180 nM or more and 113 mM or less.
(19) The differentiation inhibitor for pluripotent stem cells according to (16), wherein the ratio of the concentrations of the PKCβ inhibitor and the TNKS inhibitor comprised in the differentiation inhibitor is in the range of 167:1 or higher and 1:1600 or lower.
(20) A differentiation suppression kit for pluripotent stem cells, the differentiation suppression kit comprising the differentiation inhibitor for pluripotent stem cells according to any of (16) to (19).
(21) The method according to (1) and the differentiation inhibitor for pluripotent stem cells according to (16), wherein the PKCβ inhibitor is at least one selected from the group consisting of Go6983, GF109203X, LY-333531, Enzastaurin, Sotrastaurin, Ro-31-8220-mesylate, Ro-32-0432-hydrochloride, Go6976, Rottlerin, Midostaurin, Daphnetin, Dequalinium Chloride, Baicalein, Quercetin, Luteolin, Bisindolylmaleimide II, Calphostin C, Chelerythrine chloride, L-threo Dihydrosphingosine, and Melittin.
(22) The method according to (1) and the differentiation inhibitor for pluripotent stem cells according to (16), wherein the TNKS inhibitor is at least one selected from the group consisting of IWR-1-endo, XAV939, G007-LK, G244-LM, and WIKI4.
(23) The method according to (1) and the differentiation inhibitor for pluripotent stem cells according to (16), wherein the pluripotent stem cell is a primed pluripotent stem cell.
(24) The method according to (1), wherein the liquid medium is free of LIF.
(25) The differentiation suppression kit for pluripotent stem cells according to (20), the differentiation suppression kit including a liquid medium composition free of LIF.
(26) The method according (1), wherein the liquid medium is free of a GSK3 inhibitor.
(27) The differentiation suppression kit for pluripotent stem cells according to (20), the differentiation suppression kit including a liquid medium composition free of a GSK3 inhibitor.
(28) The method according to (1), wherein the liquid medium is free of a GSK3 inhibitor and a MEK/ERK inhibitor.
(29) The differentiation suppression kit for pluripotent stem cells according to (20), the differentiation suppression kit including a liquid medium composition free of a GSK3 inhibitor and a MEK/ERK inhibitor.
(30) The method according to (1) and the differentiation inhibitor for pluripotent stem cells according to (16), wherein the PKCβ inhibitor is a compound having the following structural formula [Formula I]: 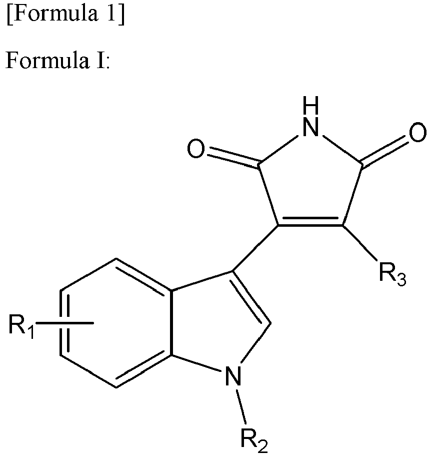 or a salt thereof, wherein
   R₁ is a hydrogen atom or an alkoxy group having 1 to 3 carbon atoms (preferably a methoxy group),
   R₂ is a hydrogen atom, an alkyl group having 1 to 3 carbon atoms (preferably a methyl group), or an alkyl group having 1 to 3 carbon atoms and substituted with -N(R_{A})₂ (preferably-(CH₂)₃-N(CH₃)₂),
   R_{A} is independently an ethyl group or a methyl group (preferably a methyl group),
   R₃ is a group represented by 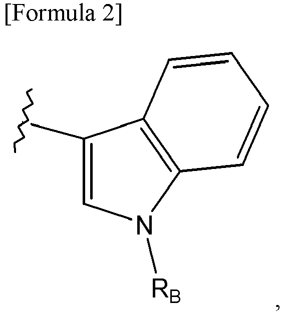 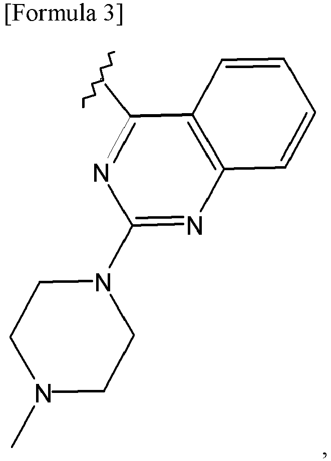 or 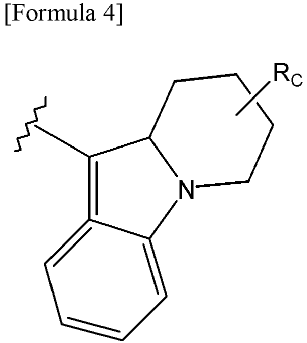
   R_{B} is a hydrogen atom, an alkyl group having 2 to 4 carbon atoms and substituted with-S-C(=NH)(-NH₂) (preferably -(CH₂)₃-S-C(=NH)(-NH₂)), or a group represented by 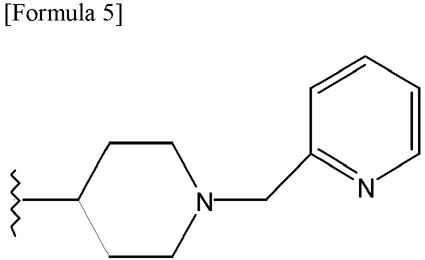
   or R₂ and R_{B} are optionally integrated together to form the following divalent group: 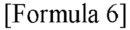 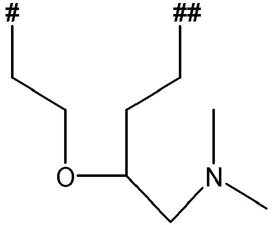 wherein # indicates bonding to the bonding position of R₂, and ## indicates bonding to the bonding position of R_{B},
   the configuration of the asymmetric carbon contained in the divalent group is preferably, but is not limited to: 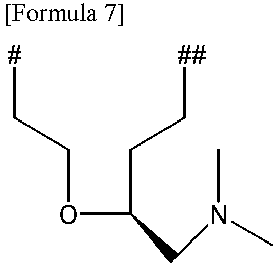
   R_{C} is an alkyl group having 1 to 3 carbon atoms and substituted with -N(R_{D})₂ (preferably -(CH₂)-N(CH₃)₂), and
   R_{D} is independently an ethyl group or a methyl group (preferably a methyl group).

Examples of the salt of the compound represented by Formula I can include a hydrochloride and a sulfate.

The present specification includes the contents disclosed in Japanese Patent Application No. 2020-021843, on the basis of which the priority of the present application is claimed.

### Advantageous Effects of Invention

The method for producing a pluripotent stem cell population according to the present invention allows a pluripotent stem cell population to be produced by suspension culture with the undifferentiated state maintained.

The differentiation inhibitor of the present invention for pluripotent stem cells allows pluripotent stem cells in suspension culture to maintain the undifferentiated state thereof.

### Brief Description of Drawings

[Figure 1] Figure 1 shows phase-contrast images acquired by photographing cell aggregates in Comparative Examples 1 to 3 and Example 1.
[Figure 2] Figure 2 shows a characteristic diagram representing results of quantitative real-time PCR analysis performed in Example 2.
[Figure 3] Figure 3 shows phase-contrast images acquired by photographing cell aggregates in Comparative Examples 4 and 5-1 to 5-4 and Examples 3-1 to 3-3.
[Figure 4] Figure 4 shows a characteristic diagram representing relative gene expression levels of PAX6 in pluripotent stem cells cultured in Reference Example 2, Comparative Examples 4 and 5-1 to 5-4, and Examples 3-1 to 3-3.
[Figure 5] Figure 5 shows a characteristic diagram representing positive rates for the undifferentiation markers OCT4, SOX2, and NANOG in pluripotent stem cells cultured in Reference Example 3, Comparative Examples 6 and 7-1 to 7-4, and Examples 5-1 and 5-2.
[Figure 6] Figure 6 shows a characteristic diagram representing relative gene expression levels of T in pluripotent stem cells cultured in Reference Example 4, Comparative Examples 8 and 9, and Example 7.
[Figure 7] Figure 7 shows a characteristic diagram representing relative gene expression levels of SOX17 in pluripotent stem cells cultured in Reference Example 4, Comparative Examples 8 and 9, and Example 7.
[Figure 8] Figure 8 shows phase-contrast images acquired by photographing cell aggregates in Comparative Example 10 and Example 9.
[Figure 9] Figure 9 shows a characteristic diagram representing relative gene expression levels of T in pluripotent stem cells cultured in Reference Example 5, Comparative Example 10, and Example 9.
[Figure 10] Figure 10 shows a characteristic diagram representing relative gene expression levels of SOX17 in pluripotent stem cells cultured in Reference Example 5, Comparative Example 10, and Example 9.
[Figure 11] Figure 11 shows a characteristic diagram representing relative gene expression levels of PAX6 in pluripotent stem cells cultured in Reference Example 5, Comparative Example 10, and Example 9.
[Figure 12] Figure 12 shows phase-contrast images of pluripotent stem cells adherent-cultured in Comparative Example 11 (under conditions with addition of a TNKS inhibitor and a PKCβ inhibitor and under conditions without addition thereof).
[Figure 13] Figure 13 shows phase-contrast images of pluripotent stem cells suspension-cultured in Example 11 (under conditions with addition of a TNKS inhibitor and a PKCβ inhibitor and under conditions without addition thereof).
[Figure 14] Figure 14 shows a characteristic diagram representing amplification efficiencies for pluripotent stem cells cultured in Comparative Example 11 and Example 11.
[Figure 15] Figure 15 shows phase-contrast images acquired by photographing pluripotent stem cells suspension-cultured in Comparative Example 12. Phase-contrast images of photographed cell aggregates.
[Figure 16] Figure 16 shows phase-contrast images acquired by photographing pluripotent stem cells suspension-cultured in Example 12. Phase-contrast images of photographed cell aggregates.
[Figure 17] Figure 17 shows characteristic diagrams representing relative gene expression levels of OCT4 and SOX2 in pluripotent stem cells cultured in Comparative Example 12 and Example 12.
[Figure 18] Figure 18 shows a characteristic diagram representing positive rates for OCT4 in pluripotent stem cells cultured in Comparative Example 12 and Example 12.
[Figure 19] Figure 19 shows a characteristic diagram representing ultimate viable cell densities of pluripotent stem cells cultured in Comparative Example 12 and Example 12.
[Figure 20] Figure 20 shows a characteristic diagram representing lactic acid concentrations in medium for pluripotent stem cells cultured in Example 12.
[Figure 21] Figure 21 shows characteristic diagrams representing relative gene expression levels of OCT4, SOX2, NANOG, T, SOX17, and PAX6 in pluripotent stem cells cultured in Example 13 and Reference Example 6.
[Figure 22] Figure 22 shows a characteristic diagram representing results of G-Band analysis for pluripotent stem cells cultured in Example 13.
[Figure 23] Figure 23 shows characteristic diagrams representing results of measurement of relative gene expression levels of CDX2, PDGFRa, SOX17, and PAX6 in pluripotent stem cells cultured in Example 13 and Reference Example 6 (maintenance of undifferentiation) and Example 14 and Reference Example 7 (induction of differentiation).

### Description of Embodiments

### 1. Differentiation inhibitor for Pluripotent Stem Cells

### 1-1. Summary

The differentiation inhibitor for pluripotent stem cells according to the present invention is characterized by containing a protein kinase Cβ (PKCβ) inhibitor and a tankyrase (TNKS) inhibitor. The differentiation inhibitor for pluripotent stem cells according to the present invention can be a composition containing a PKCβ inhibitor and a TNKS inhibitor. The differentiation inhibitor for pluripotent stem cells according to the present invention allows a pluripotent stem cell population to be produced with ease by suspension culture with cells in the suspension culture maintained in an undifferentiated state.

### 1-2. Definition of Terms

The following terms to be used in the present specification will be defined.

### <<Cells>>

A "pluripotent stem cell" as a subject matter of the invention in the present specification refers to a cell having pluripotent capacity (pluripotency) to differentiate into all types of cells constituting a living body, and being capable of permanently continuing proliferation with the pluripotency maintained in *in vitro* culture under proper conditions. More specifically, pluripotency means an ability to differentiate into germ layers constituting an individual (for vertebrates, three germ layers: ectoderm, mesoderm, and endoderm). Examples of such cells include embryonic stem cells (ES cells), embryonic germ cells (EG cells), germline stem cells (GS cells), and induced pluripotent stem cells (iPS cells). An "ES cell" is a pluripotent stem cell prepared from an early embryo. An "EG cell" is a pluripotent stem cell prepared from a fetal primordial germ cell (Shamblott M. J. et al., 1998, Proc. Natl. Acad. Sci. USA., 95:13726-13731). A "GS cell" is a pluripotent stem cell prepared from a testicular cell (Conrad S., 2008, Nature, 456:344-349). An "iPS cell" refers to a pluripotent stem cell that has become reprogrammable by introducing a few genes encoding initialization factors into a differentiated somatic cell to turn the somatic cell into an undifferentiated state.

The pluripotent stem cells in the present specification can be cells derived from a multicellular organism. The pluripotent stem cells are preferably cells derived from an animal, and more preferably cells derived from a mammal. Examples of the mammal include a rodent such as a mouse, a rat, a hamster, and a guinea pig, a domestic or pet animal such as a dog, a cat, a rabbit, a bovine, a horse, sheep, and a goat, and a primate such as a human, a rhesus monkey, a gorilla, and a chimpanzee. Particularly preferred are cells derived from a human.

The pluripotent stem cells to be used in the present specification include naive pluripotent stem cells and primed pluripotent stem cells. By definition, a naive pluripotent stem cell is in a state with pluripotency close to that found in the preimplantation inner cell mass, and a primed pluripotent stem cell is in a state with pluripotency close to that found in the postimplantation epiblast. As compared with naive pluripotent stem cells, primed pluripotent stem cells are characterized by less frequent contribution to ontogenesis, X-chromosome transcription activity found only for one chromatid, and high-level transcription-suppressive histone modification. A marker gene for primed pluripotent stem cells is OTX2, and marker genes for primed pluripotent stem cells are REX1 and the KLF family. Primed pluripotent stem cells form flat colonies, and naive pluripotent stem cells form dome-shaped colonies. In particular, it is preferable to use primed pluripotent stem cells for the pluripotent stem cells to be used in the present specification.

Commercially available cells or donated cells, or newly prepared cells may be used for the pluripotent stem cells to be used in the present specification. In use for the invention of the present specification, the pluripotent stem cells are preferably, but not limited to, iPS cells or ES cells.

If a commercially available product is used for the iPS cells to be used in the present specification, applicable commercially available products include, but are not limited to, 253G1 strain, 253G4 strain, 201B6 strain, 201B7 strain, 409B2 strain, 454E2 strain, 606A1 strain, 610B1 strain, 648A1 strain, HiPS-RIKEN-1A strain, HiPS-RIKEN-2A strain, HiPS-RIKEN-12A strain, Nips-B2 strain, TkDN4-M strain, TkDA3-1 strain, TkDA3-2 strain, TkDA3-4 strain, TkDA3-5 strain, TkDA3-9 strain, TkDA3-20 strain, hiPSC38-2 strain, MSC-iPSC1 strain, BJ-iPSC1 strain, RPChiPS771-2, WTC-11 strain, 1231A3 strain, 1383D2 strain, 1383D6 strain, 1210B2 strain, 1201C1 strain, and 1205B2 strain.

If the iPS cells to be used in the present specification are newly prepared cells, applicable combinations of genes for initialization factors to be introduced include, but are not limited to, a combination of the OCT3/4 gene, KLF4 gene, SOX2 gene, and c-Myc gene (Yu J, et al. 2007, Science, 318:1917-20.) and a combination of the OCT3/4 gene, SOX2 gene, LIN28 gene, and Nanog gene (Takahashi K, et al. 2007, Cell, 131:861-72.). Any mode may be employed for introducing those genes into cells, without limitation; for example, transfection with plasmids or introduction of synthetic RNA may be employed, and proteins formed therefrom may be introduced. Alternatively, iPS cells prepared with a method involving use of microRNA, RNA, a small-molecule compound, or the like may be used. Moreover, newly prepared clinical-grade iPS cells may be used.

If a commercially available product is used for the ES cells to be used in the present specification, applicable commercially available products include, but are not limited to, KhES-1 strain, KhEs-2 strain, KhEs-3 strain, KhEs-4 strain, KhEs-5 strain, SEES1 strain, SEES2 strain, SEES3 strain, SEES-4 strain, SEEs-5 strain, SEEs-6 strain, SEEs-7 strain, HUES8 strain, CyT49 strain, H1 strain, H9 strain, and HS-181 strain.

### «Cell Aggregate»

In the present specification, a "cell aggregate" is a massive cell population formed through cell aggregation in suspension culture, and is also called a spheroid. Cell aggregates are generally spherical in typical cases. Cells constituting a cell aggregate are not limited as long as they are one or more types of the aforementioned cells. For example, a cell aggregate composed of pluripotent stem cells such as human pluripotent stem cells and human embryonic stem cells include cells expressing a pluripotent stem cell marker and/or being positive for a pluripotent stem cell marker.

Pluripotent stem cell markers are gene markers specifically or excessively expressed in pluripotent stem cells, and examples thereof can include Alkaline Phosphatase, Nanog, OCT4, SOX2, TRA-1-60, c-Myc, KLF4, LIN28, SSEA-4, and SSEA-1.

Pluripotent stem cell markers can be detected with any detection method in the art. Examples of methods for detecting cell markers include, but are not limited, flow cytometry. In the case that a fluorescence-labeled antibody is used as a detection reagent in flow cytometry, if a cell emitting more intense fluorescence than a negative control (isotype control) is detected, the cell is determined as "positive" for the marker. The proportion of cells positive for a fluorescence-labeled antibody as analyzed by flow cytometry is occasionally referred to as the "positive rate". Any antibody known in the art can be used for such fluorescence-labeled antibodies, and examples thereof include, but are not limited to, antibodies labeled with fluorescein isothiocyanate (FITC), phycoerythrin (PE), or allophycocyanin (APC).

If cells constituting a cell aggregate are pluripotent stem cells, the positive rate for a pluripotent stem cell marker can be preferably 80% or higher, more preferably 90% or higher, more preferably 91% or higher, more preferably 92% or higher, more preferably 93% or higher, more preferably 94% or higher, more preferably 95% or higher, more preferably 96% or higher, more preferably 97% or higher, more preferably 98% or higher, more preferably 99% or higher, and more preferably 100% or lower. Cell aggregates in which the percentage of cells expressing a pluripotent stem cell marker and/or being positive for a pluripotent stem cell marker falls within the range are highly undifferentiated, highly homogeneous cell populations.

### <<Culture and Medium>>

"Suspension culture", one of cell culture methods, refers to allowing cells in a suspended state in medium to proliferate. The "suspended state" in the present specification refers to a state in which cells are not adhering to an external matrix such as a culture vessel. A "suspension culture method" is a method of suspension-culturing cells, and cells in this method are present as a cell cluster formed through aggregation in culture solution. An adherent culture method is a counterpart culture method to the suspension culture method. The "adherent culture method" is a method of adherent-culturing cells. "Adherent culture" refers to allowing cells to proliferate as a monolayer in principle with the cells adhered to an external matrix or the like such as a culture vessel. In normal cases, the adherent cells can be cultured not only through adherent culture but also through suspension culture.

In the present invention, "medium" refers to a liquid or solid substance prepared for culturing cells. In principle, medium contains components indispensable for proliferation and/or maintenance of cells over their minimum requirements. Unless otherwise stated, liquid medium for animal cells for use in culture of cells derived from an animal is employed as medium in the present specification.

In the present specification, "minimal essential medium" refers to medium that serves as a base for media for various animal cells. Even minimal essential medium alone is applicable to culture, and media specific to different cells for different purposes may be prepared by adding various culture additives. Examples of minimal essential medium applicable in the present specification include, but are not limited to, BME medium, BGJb medium, CMRL1066 medium, Glasgow MEM medium, Improved MEM Zinc Option medium, IMDM medium (Iscove'S Modified Dulbecco'S Medium), Medium 199 medium, Eagle MEM medium, αMEM medium, DMEM medium (Dulbecco'S Modified Eagle'S Medium), Ham's F10 medium, Ham's F12 medium, RPMI 1640 medium, Fischer's medium, and mixed media of them (e.g., DMEM/F12 medium (Dulbecco'S Modified Eagle'S Medium/Nutrient Mixture F-12 Ham)). For DMEM/F12 medium, in particular, medium obtained by mixing DMEM medium and Ham's F12 medium at a weight ratio preferably in the range of 60/40 or higher and 40/60 or lower, such as 58/42, 55/45, 52/48, 50/50, 48/52, 45/55, and 42/58, is used. In addition, other media used for culture of human iPS cells and human ES cells can be preferably used. Medium to be used in the present invention is preferably medium containing no serum, in other words, serum-free medium.

In the present specification, a "culture additive" is a substance that is added to medium for culture, except serum. Specific examples of culture additives include, but are not limited to, L-ascorbic acid, insulin, transferrin, selenium, sodium hydrogen carbonate, growth factors, fatty acid or lipid, amino acids (e.g., non-essential amino acids), vitamins, cytokine, antioxidants, 2-mercaptoethanol, pyruvic acid, buffering agents, inorganic salts, and antibiotics. The insulin, transferrin, and cytokine may be naturally occurring ones separated from tissue or serum or the like of an animal (preferably, human, mouse, rat, bovine, horse, goat, etc.), or recombinant proteins produced with a gene engineering technique. Examples of applicable growth factors include, but are not limited to, FGF2 (Basic fibroblast growth factor-2), TGF-β1 (Transforming growth factor-β1), Activin A, IGF-1, MCP-1, IL-6, PAI, PEDF, IGFBP-2, LIF, and IGFBP-7. Examples of applicable antibiotics include, but are not limited to, penicillin, streptomycin, and amphotericin B. FGF2 and/or TGF-β1 is a particularly preferable growth factor as a culture additive for medium to be used in the present invention.

It is preferable that each medium contain a ROCK inhibitor. Examples of ROCK inhibitors include Y-27632. Cell death in suspension culture of pluripotent stem cells can be significantly reduced by inclusion of a ROCK inhibitor in medium.

If primed pluripotent stem cells are to be cultured, it is preferable that each medium have a composition free of LIF. If primed pluripotent stem cells are to be cultured, it is preferable to employ a medium composition free of any one of a GSK3 inhibitor and a MEK/ERK inhibitor, preferably of both of them. Such medium free of LIF, a GSK3 inhibitor, and a MEK/ERK inhibitor allows primed pluripotent stem cells to be cultured with the undifferentiated state maintained, without causing conversion into the naive state.

Medium to be used in the present invention can contain one or more of the culture additives. Medium to which the culture additives are to be added is typically any of the minimal essential media, but is not limited thereto.

The culture additives in the form of a solution, derivatives, salts, or a mixed reagent or the like can be added to medium. For example, L-ascorbic acid in the form of a derivative such as magnesium ascorbyl-2-phosphate may be added to medium, and selenium in the form of a selenite (such as sodium selenite) may be added to medium. Insulin, transferrin, and selenium in the form of an ITS reagent (insulin-transferrin-selenium) can be added to medium. Commercially available medium to which at least one selected from L-ascorbic acid, insulin, transferrin, selenium, and sodium hydrogen carbonate may be used. Examples of commercially available medium to which insulin and transferrin have been added include CHO-S-SFM II (Life Technologies Japan Ltd.), Hybridoma-SFM (Life Technologies Japan Ltd.), eRDF Dry Powdered Media (Life Technologies Japan Ltd.), UltraCULTURE (TM) (BioWhittaker), UltraDOMA (TM) (BioWhittaker), UltraCHO (TM) (BioWhittaker), UltraMDCK (TM) (BioWhittaker), STEMPRO (R) hESC SFM (Life Technologies Japan Ltd.), Essential8 (TM) (Life Technologies Japan Ltd.), StemFit (R) AK02N (Ajinomoto Co., Inc.), mTeSR1 (VERITAS Corporation), and TeSR2 (VERITAS Corporation).

The most preferable medium to be used in the present invention is serum-free medium containing L-ascorbic acid, insulin, transferrin, selenium, and sodium hydrogen carbonate, and at least one growth factor. Particularly preferred is DMEM/F12 medium containing L-ascorbic acid, insulin, transferrin, selenium, and sodium hydrogen carbonate, and at least one growth factor (preferably FGF2 and TGF-β1), and being free of serum.

### <<PKCβ Inhibitor>>

In the present specification, the term "protein kinase Cβ (PKCβ) inhibitor" means a substance that inhibits or suppresses the activity of PKCβ. Protein kinases each have a catalytic region in the C-terminal side and a regulatory region in the N-terminal side. The catalytic region is composed of a sequence that recognizes phosphorylated residues on a substrate protein and a sequence that forms an ATP/Mg²⁺ bond. The regulatory region is composed of C1 and C2 domains.

PKC includes PKCα, PKCβI, PKCβII, and PKCγ as conventional isozymes. PKC includes PKCδ, PKCε, PKCθ, and PKCη as novel isozymes, and PKCζ, PKCλ, and PKCµ as atypical isozymes.

In the present specification, the term PKCβ means both of those PKCβI and PKCβII, or one of PKCβI and PKCβII. In the present specification, the term PKCβ inhibitor means a substance that inhibits at least PKCβI and/or PKCβII among those conventional, novel, and atypical isozymes. That is, the term PKCβ inhibitor means any of a substance that inhibits or suppresses only the activity of PKCβI, a substance that inhibits or suppresses only the activity of PKCβII, and a substance that inhibits or suppresses the activities of PKCβI and PKCβII.

The PKCβ inhibitor may be a substance that specifically inhibits or suppresses only the activity of PKCβ, and may be a substance that inhibits or suppresses the activity of another isozyme in addition to that of PKCβI or PKCβII. For example, the PKCβ inhibitor may be a substance that inhibits or suppresses the activities of all the aforementioned conventional, novel, and atypical isozymes including PKCβI and PKCβII. The PKCβ inhibitor may be a substance that inhibits or suppresses the activities of the conventional isozymes PKCα and PKCγ in addition to those of PKCβI and PKCβII. Moreover, the PKCβ inhibitor may be a substance that inhibits or suppresses the activities of the novel isozymes PKCδ, PKCε, PKCθ, and PKCη in addition to those of PKCβI and PKCβII.

Examples of the PKCβ inhibitor include a compound that directly or indirectly acts on PKCβ, an antisense nucleic acid for a gene encoding PKCβ, an RNA-interference-inducible nucleic acid (e.g., siRNA), a dominant-negative mutant, and an expression vector for any of them.

An example of the PKCβ inhibitor can be a compound having the following structural formula [Formula I].

A compound represented by: 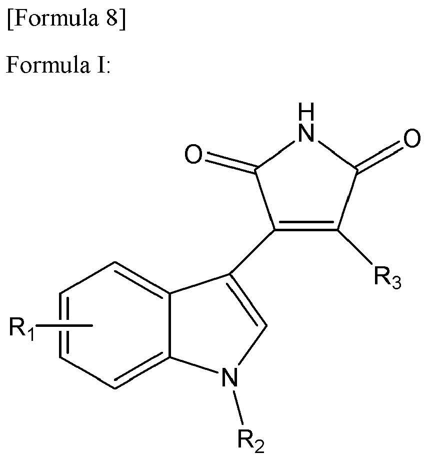 or a salt thereof.

In Formula I,
R₁ is a hydrogen atom or an alkoxy group having 1 to 3 carbon atoms (preferably a methoxy group), R₂ is a hydrogen atom, an alkyl group having 1 to 3 carbon atoms (preferably a methyl group), or an alkyl group having 1 to 3 carbon atoms and substituted with -N(R_{A})₂ (preferably -(CH₂)₃-N(CH₃)₂),
R_{A} is independently an ethyl group or a methyl group (preferably a methyl group),
R₃ is a group represented by 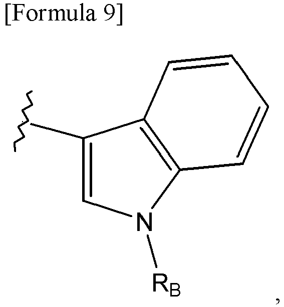 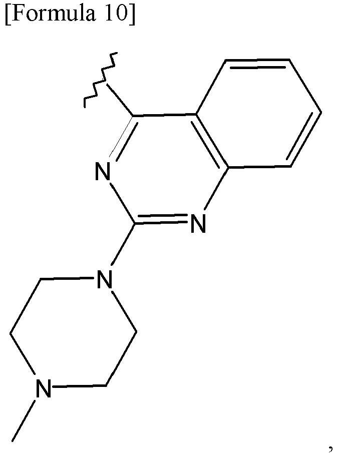 or 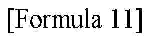 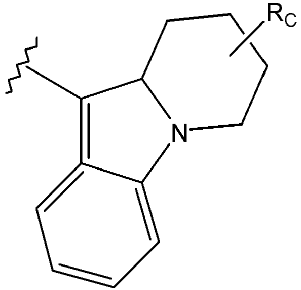
R_{B} is a hydrogen atom, an alkyl group having 2 to 4 carbon atoms and substituted with-S-C(=NH)(-NH₂) (preferably -(CH₂)₃-S-C(=NH)(-NH₂)), or a group represented by 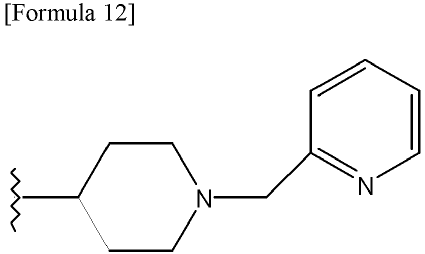 or R₂ and R_{B} are optionally integrated together to form the following divalent group: 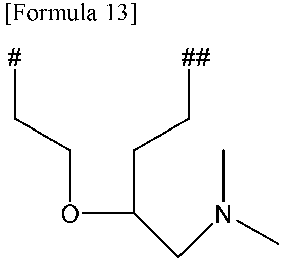 wherein # indicates bonding to the bonding position of R₂, and ## indicates bonding to the bonding position of R_{B},
the configuration of the asymmetric carbon contained in the divalent group is preferably, but is not limited to: 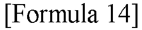 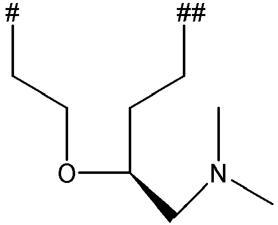
R_{C} is an alkyl group having 1 to 3 carbon atoms and substituted with -N(R_{D})₂ (preferably -(CH₂)-N(CH₃)₂), and
R_{D} is independently an ethyl group or a methyl group (preferably a methyl group).

Examples of the salt of the compound represented by Formula I can include a hydrochloride and a sulfate.

Specific examples of the PKCβ inhibitor having the above structural formula [Formula I] include a compound selected from the group consisting of Go6983, GF109203X, LY-333531, Enzastaurin, Sotrastaurin, Ro-31-8220-mesylate, Ro-32-0432-hydrochloride, Go6976, Rottlerin, Midostaurin, Daphnetin, Dequalinium Chloride, Baicalein, Quercetin, Luteolin, Bisindolylmaleimide II, Calphostin C, Chelerythrine chloride, L-threo Dihydrosphingosine, and Melittin. Among the PKCβ inhibitors having the above structural formula, a compound selected from the group consisting of Go6983, GF109203X, and LY-333531 is preferably used.

The structural formula of Go6983 (3-[1-[3-(dimethylamino)propyl]-5-methoxy-1H-indol-3-yl]-4-(1H-indol-3-yl)-1H-pyrrole-2,5-dione) is shown in the following.

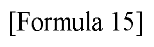

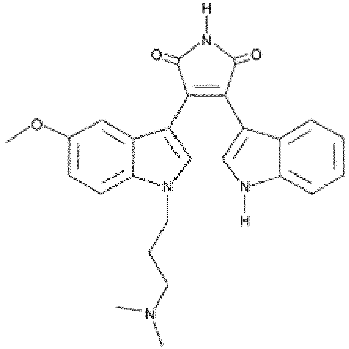

The structural formula of GF109203X (2-[1-(3-dimethylaminopropyl)indol-3-yl]-3-(indol-3-yl)maleimide) is shown in the following.

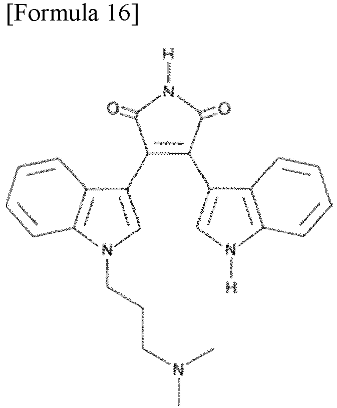

The structural formula of LY-333531 ((9S)-[(dimethylamino)methyl]-6,7,10,11-tetrahydro-9H,18H-5,21:12,17-dimethenodibenzo[e,k]pyrrolo[3,4-h][1,4,13]oxsadiazacyclohexadecine-18,20(19H)-dione, monohydroxychloride) is shown in the following.

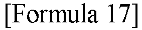

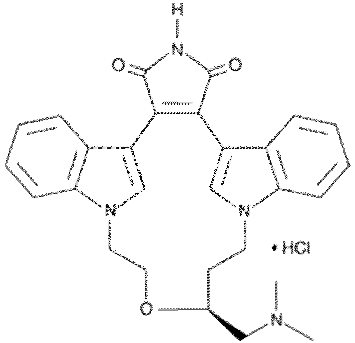

The structural formula of Enzastaurin (3-(1-methylindol-3-yl)-4-[1-[1-(pyridin-2-ylmethyl)piperidin-4-yl]indol-3-yl]pyrrole-2,5-dione) is shown in the following.

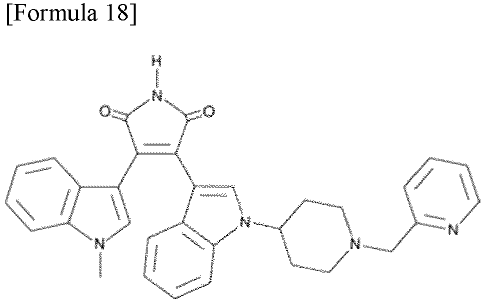

The structural formula of Sotrastaurin (3-(1H-indol-3-yl)-4-(2-(4-methylpiperazin-1-yl)quinazolin-4-yl)-1H-pyrrole-2,5-dione) is shown in the following.

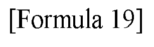

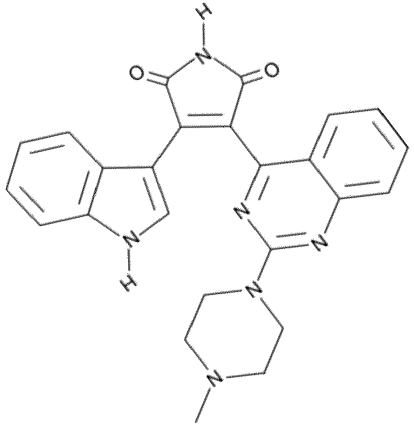

The structural formula of Ro-31-8220-mesylate (3-[3-[2,5-dihydro-4-(1-methyl-1H-indol-3-yl)-2,5-dioxo-1H-pyrrole-3-yl]-1H-indol-1-yl]propyl carbamimidothioate mesylate) is shown in the following.

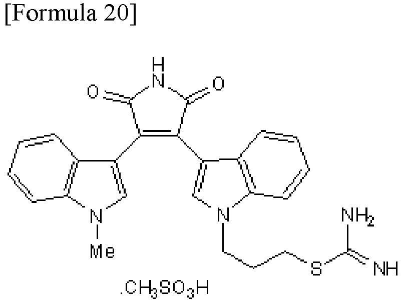

The structural formula of Ro-32-0432-hydrochloride (3-[(8S)-8-[(dimethylamino)methyl]-6,7,8,9-tetrahydropyrido[1,2-a]indol-10-yl]-4-(1-methyl-1H-indol-3-yl)-1H-pyrrole-2,5-dione hydrochloride) is shown in the following.

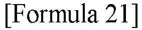

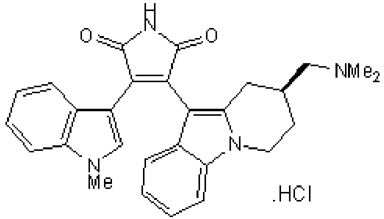

### <<TNKS Inhibitor>>

In the present specification, the term "tankyrase (TNKS) inhibitor" means a substance that inhibits or suppresses the activity of tankyrase. Tankyrase belongs to the poly(ADP-ribose) polymerase (PARP) family to poly(ADP-ribosylate) a target protein, and tankyrase 1 (tankyrase-1/PARP-5a) and tankyrase 2 (tankyrase-2/PARP-5b) are known. Tankyrase is known to have a function to promote the telomere elongation by telomerase through poly(ADP-ribosylation) of the telomere protein TRF1 to liberate it from a telomere.

In the present specification, the term TNKS means both of those tankyrase 1 and tankyrase 2, or one of tankyrase 1 and tankyrase 2. In the present specification, the term TNKS inhibitor means a substance that inhibits tankyrase 1 and/or tankyrase 2. That is, the term TNKS inhibitor means any of a substance that inhibits or suppresses only the activity of tankyrase 1, a substance that inhibits or suppresses only the activity of tankyrase 2, and a substance that inhibits or suppresses the activities of tankyrase 1 and tankyrase 2.

Examples of the TNKS inhibitor include a compound that directly or indirectly acts on TNKS, an antisense nucleic acid for a gene encoding TNKS, an RNA-interference-inducible nucleic acid (e.g., siRNA), a dominant-negative mutant, and an expression vector for any of them.

An example of the TNKS inhibitor can be a compound selected from the group consisting of IWR-1-endo, XAV939, G007-LK, G244-LM, MSC2504877, and WIKI4. Among the TNKS inhibitors, IWR-1-endo and/or XAV939 is particularly preferably used.

The structural formula of IWR-1-endo (4-(1,3,3a,4,7,7a-hexahydro-1,3-dioxo-4,7-methano-2H-isoindol-2-yl)-N-8-quinolinyl-benzamide) is shown in the following.

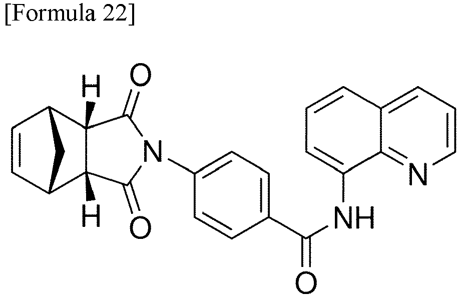

The structural formula of XAV939 (3,5,7,8-tetrahydro-2-[4-(trifluoromethyl)phenyl]-4H-thiopyrano[4,3-d]pyrimidin-4-one) is shown in the following.

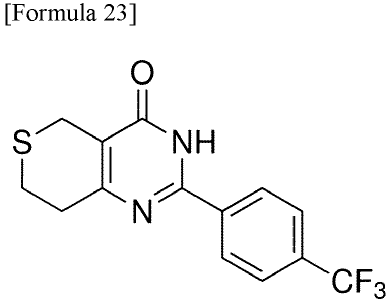

The structural formula of G007-LK (4-[5-[(1E)-2-[4-(2-chlorophenyl)-5-[5-(methylsulfonyl)-2-pyridinyl]-4H-1,2,4-thiazol-3-yl]ethenyl]-1,3,4-oxsadiazol-2-yl]-benzonitrile) is shown in the following.

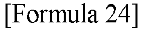

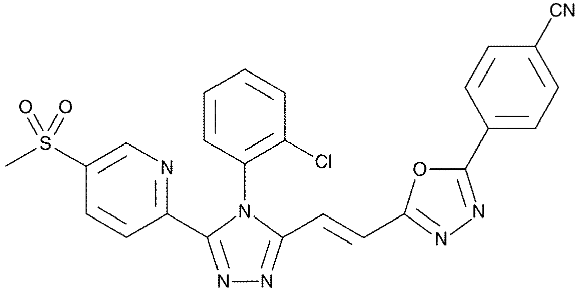

The structural formula of G244-LM (3,5,7,8-tetrahydro-2-[4-[2-(methylsulfonyl)phenyl]-1-piperazinyl]-4H-thiopyrano[4,3-d]pyrimidin-4-one) is shown in the following.

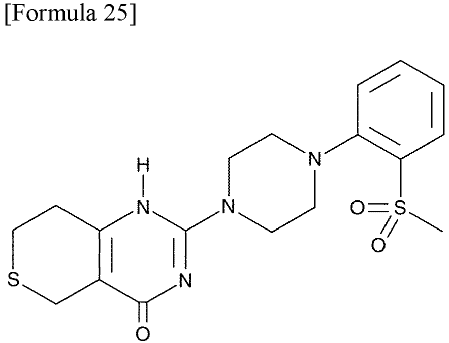

The structural formula of WIKI4 (2-[3-[[4-(4-methoxyphenyl)-5-(4-pyridinyl)-4H-1,2,4-thiazol-3-yl]thio]propyl]-1H-benzo[de]isoquinoline-1,3(2H)-dione) is shown in the following.

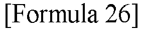

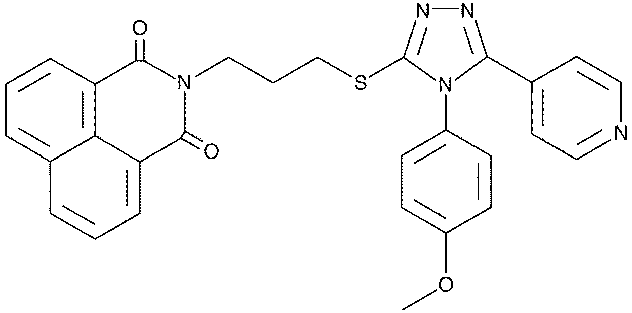

### 1-3. Configuration

The differentiation inhibitor for pluripotent stem cells according to the present invention comprises a PKCβ inhibitor and a TNKS inhibitor, which have been defined above in 1-2., as active ingredients.

Here, the PKCβ inhibitor in the differentiation inhibitor for pluripotent stem cells may be one PKCβ inhibitor or a combination of two or more different PKCβ inhibitors.

The lower limit of the concentration of the PKCβ inhibitor is not limited, and can be determined with considering the range that gives differentiation-suppressing effect on pluripotent stem cells.

The final concentration of the PKCβ inhibitor in the liquid medium can be, for example, 25 nM or more, 30 nM or more, 50 nM or more, 80 nM or more, 100 nM or more, 150 nM or more, 200 nM or more, 500 nM or more, 700 nM or more, 1 µM or more, 3 µM or more, 5 µM or more, and 10 µM or more.

The upper limit of the concentration of the PKCβ inhibitor is not limited, and can be determined with considering the range that gives differentiation-suppressing effect on pluripotent stem cells, the solubility of the PKCβ inhibitor, and others.

The final concentration of the PKCβ inhibitor in the liquid medium can be, for example, 15 µM or less, 10 µM or less, 5 µM or less, 3 µM or less, 1 µM or less, 700 nM or less, 500 nM or less, 200 nM or less, 150 nM or less, 100 nM or less, 80 nM or less, 50 nM or less, and 30 nM or less.

In the case that the differentiation inhibitor for pluripotent stem cells is a composition, the concentration of the PKCβ inhibitor in the composition can be determined so as to fall within the above range when the composition is added to medium. If the composition is 2-fold diluted for use, for example, the lower limit of the PKCβ inhibitor in the composition can be 50 nM or more, 60 nM or more, 100 nM or more, 160 nM or more, 200 nM or more, 300 nM or more, 400 nM or more, 1 µM or more, 1.4 µM or more, 2 µM or more, 6 µM or more, 10 µM or more, and 20 µM or more.

The upper limit of the PKCβ inhibitor in the composition is not limited, and can be the solubility of the PKCβ inhibitor. Specifically, the upper limit of the PKCβ inhibitor in the composition can be 200 mM.

The TNKS inhibitor in the differentiation inhibitor for pluripotent stem cells may be one TNKS inhibitor or a combination of two or more different TNKS inhibitors.

The lower limit of the concentration of the TNKS inhibitor is not limited, and can be determined with considering the range that gives differentiation-suppressing effect on pluripotent stem cells.

The final concentration of the TNKS inhibitor in the liquid medium can be, for example, 90 nM or more, 100 nM or more, 150 nM or more, 200 nM or more, 300 nM or more, 400 nM or more, 500 nM or more, 600 nM or more, 700 nM or more, 800 nM or more, 900 nM or more, 1 µM or more, 1.5 µM or more, 3 µM or more, 5 µM or more, 10 µM or more, 15 µM or more, 30 µM or more, and 35 µM or more.

The upper limit of the concentration of the TNKS inhibitor is not limited, and can be determined with considering the range that gives differentiation-suppressing effect on pluripotent stem cells, the solubility of the TNKS inhibitor, and others.

The final concentration of the TNKS inhibitor in the liquid medium can be, for example, 40 µM or less, 35 µM or less, 30 µM or less, 15 µM or less, 10 µM or less, 5 µM or less, 3 µM or less, 1.5 µM or less, 1 µM or less, 900 nM or less, 800 nM or less, 700 nM or less, 600 nM or less, 500 nM or less, 400 nM or less, 300 nM or less, 200 nM or less, 150 nM or less, and 100 nM or less.

In the case that the differentiation inhibitor for pluripotent stem cells is a composition, the concentration of the TNKS inhibitor in the composition can be determined so as to fall within the above range when the composition is added to medium. If the composition is 2-fold diluted for use, for example, the lower limit of the TNKS inhibitor can be 180 nM or more, 200 nM or more, 300 nM or more, 400 nM or more, 600 nM or more, 800 nM or more, 1 µM or more, 1.2 µM or more, 1.4 µM or more, 1.6 µM or more, 1.8 µM or more, 2 µM or more, 3 µM or more, 6 µM or more, 10 µM or more, 20 µM or more, 30 µM or more, 60 µM or more, and 70 µM or more.

The upper limit of the TNKS inhibitor in the composition is not limited, and can be the solubility of the TNKS inhibitor. Specifically, the upper limit of the TNKS inhibitor in the composition can be 113 mM.

The lower limit of the ratio of the concentrations of the PKCβ inhibitor and the TNKS inhibitor comprised in the liquid medium is not limited, and can be, for example, 167:1 or higher, 111:1 or higher, 56:1 or higher, 33:1 or higher, 11:1 or higher, 7.8:1 or higher, 5.6:1 or higher, 2.2:1 or higher, 1.7:1 or higher, or 1.1:1 or higher. The upper limit of the ratio of the concentrations of the PKCβ inhibitor and the TNKS inhibitor comprised in the liquid medium is not limited, and can be, for example, 1:1600 or lower, 1:1400 or lower, 1:1200 or lower, 1:600 or lower, 1:400 or lower, 1:200 or lower, 1:120 or lower, 1:60 or lower, 1:40 or lower, 1:36 or lower, 1:32 or lower, 1:28 or lower, 1:24 or lower, 1:20 or lower, 1:16 or lower, 1:12 or lower, 1:8 or lower, 1:6 or lower, or 1:4 or lower. The ratio of the concentrations of the PKCβ inhibitor and the TNKS inhibitor comprised in the liquid medium is not limited, and can be, for example, in the range of 167:1 or higher and 1:1600 or lower. The ratio of the concentrations of the PKCβ inhibitor and the TNKS inhibitor comprised in the liquid medium can be in the range of 111:1 or higher and 1:1600 or lower, in the range of 56:1 or higher and 1:1600 or lower, in the range of 33:1 or higher and 1:1600 or lower, in the range of 11:1 or higher and 1:1600 or lower, in the range of 7.8:1 or higher and 1:1600 or lower, in the range of 5.6:1 or higher and 1:1600 or lower, in the range of 2.2:1 or higher and 1:1600 or lower, in the range of 1.7:1 or higher and 1:1600 or lower, and in the range of 1.1:1 or higher and 1:1600 or lower. Moreover, the ratio of the concentrations of the PKCβ inhibitor and the TNKS inhibitor comprised in the liquid medium can be in the range of 167:1 or higher and 1:1400 or lower, in the range of 167:1 or higher and 1:1200 or lower, in the range of 167:1 or higher and 1:600 or lower, in the range of 167:1 or higher and 1:400 or lower, in the range of 167:1 or higher and 1:200 or lower, in the range of 167:1 or higher and 1:120 or lower, in the range of 167:1 or higher and 1:60 or lower, in the range of 167:1 or higher and 1:40 or lower, in the range of 167:1 or higher and 1:36 or lower, in the range of 167:1 or higher and 1:32 or lower, in the range of 167:1 or higher and 1:28 or lower, in the range of 167:1 or higher and 1:24 or lower, in the range of 167:1 or higher and 1:20 or lower, in the range of 167:1 or higher and 1:16 or lower, in the range of 167:1 or higher and 1:12 or lower, in the range of 167:1 or higher and 1:8 or lower, in the range of 167:1 or higher and 1:6 or lower, and in the range of 167:1 or higher and 1:4 or lower.

Examples of additional components combined with the PKCβ inhibitor and the TNKS inhibitor as active ingredients in the case that the differentiation inhibitor for pluripotent stem cells is a composition include a carrier. The carrier contains a solvent and/or an excipient.

Examples of the solvent include water, buffers (including PBS), physiological saline, and organic solvents (DMSO, DMF, xylene, lower alcohols).

Examples of the excipient include antibiotics, buffering agents, thickeners, coloring agents, stabilizers, surfactants, emulsifying agents, antiseptics, preservatives, and antioxidants. Applicable antibiotics include, but are not limited to, penicillin, streptomycin, and amphotericin B. Examples of buffering agents include phosphate buffer, Tris-hydrochloric acid buffer, and glycine buffer. Examples of thickeners include gelatin and polysaccharides. Examples of coloring agents include phenol red. Examples of stabilizers include albumin, dextran, methylcellulose, and gelatin. Examples of surfactants include cholesterol, alkyl glycoside, alkyl polyglucoside, alkyl monoglyceryl ether, glucoside, maltoside, neopentyl glycols, polyoxyethylene glycols, thioglucoside, thio maltoside, peptide, saponin, phospholipid, fatty acid sorbitan ester, and fatty acid diethanolamide. Examples of emulsifying agents include glycerin fatty acid ester, sorbitan fatty acid ester, propylene glycol fatty acid ester, and sucrose fatty acid ester. Examples of antiseptics include aminoethylsulfonic acid, benzoic acid, sodium benzoate, ethanol, sodium edetate, agar, dl-camphor, citric acid, sodium citrate, salicylic acid, sodium salicylate, phenyl salicylate, dibutylhydroxytoluene, sorbic acid, potassium sorbate, nitrogen, dehydroacetic acid, sodium dehydroacetate, 2-naphthol, white soft sugar, honey, isobutyl para-hydroxybenzoate, isopropyl para-hydroxybenzoate, ethyl para-hydroxybenzoate, butyl para-hydroxybenzoate, propyl para-hydroxybenzoate, methyl para-hydroxybenzoate, 1-menthol, and Eucalyptus oil. Examples of preservatives include benzoic acid, sodium benzoate, ethanol, sodium edetate, dried sodium sulfite, citric acid, glycerin, salicylic acid, sodium salicylate, digutylhydroxytoluene, D-sorbitol, sorbic acid, potassium sorbate, sodium dehydroacetate, isobutyl para-hydroxybenzoate, isopropyl para-hydroxybenzoate, ethyl para-hydroxybenzoate, butyl para-hydroxybenzoate, propyl para-hydroxybenzoate, methyl para-hydroxybenzoate, propylene glycol, and phosphoric acid. Examples of antioxidants include citric acid, citric acid derivatives, vitamin C and derivatives thereof, lycopene, vitamin A, carotenoids, vitamin B and derivatives thereof, flavonoids, polyphenols, glutathione, selenium, sodium thiosulfate, vitamin E and derivatives thereof, α-lipoic acid and derivatives thereof, pycnogenol, flavangenol, superoxide dismutase (SOD), glutathione peroxidase, glutathione-S-transferase, glutathione reductase, catalase, ascorbate peroxidase, and mixtures of them.

The differentiation inhibitor for pluripotent stem cells may contain one or more growth factors. Examples of the growth factors include FGF2 and TGF-β1.

The form of the differentiation inhibitor for pluripotent stem cells may be any of liquids and solids (including granules, powders, and dust formulations). The form of application of the differentiation inhibitor for pluripotent stem cells is not limited. For example, the differentiation inhibitor for pluripotent stem cells may be in the form of medium to be used for suspension culture, or in the form of an additive to be blended in preparation of medium for suspension culture.

### 1-4. Advantageous Effects

The differentiation inhibitor of the present invention for pluripotent stem cells allows pluripotent stem cells to form a cell aggregate with the undifferentiated state of the pluripotent stem cells maintained in a suspension culture system. This enables efficient mass production of pluripotent stem cells.

### 2. Method for Producing Pluripotent Stem Cell Population

### 2-1. Summary

The method for producing a pluripotent stem cell population according to the present invention comprises the step of performing suspension culture of a pluripotent stem cell in the presence of a protein kinase Cβ (PKCβ) inhibitor and a tankyrase (TNKS) inhibitor. With the method for producing a pluripotent stem cell population according to the present invention, the presence of the PKCβ inhibitor and the TNKS inhibitor allows a pluripotent stem cell population to be produced with ease by suspension culture with the undifferentiated state of pluripotent stem cells maintained.

### 2-2. Method

The method of the present aspect includes: a suspension culture step as an essential step; and a maintenance culture step and a collection step as optional steps. These steps will be described in the following.

### 2-2-1. Maintenance Culture Step

The "maintenance culture step" is a step of culture for allowing a cell population before the suspension culture step, or a cell aggregate obtained after the suspension culture step or after the subsequent collection step to proliferate with an undifferentiated state maintained. An animal cell culture method known in the art can be used for the maintenance culture. For example, the maintenance culture may be adherent culture, in which cells are cultured with the cells adhered to a culture substrate such as a vessel and a carrier, or suspension culture.

Examples of the present step and an animal cell culture method to be used in the suspension culture step described later will be shown for description, though they are not limited to the examples.

### (Cells)

The cells to be used in the present step are cells capable of cell aggregation in suspension culture. As described in the subsection "Culture and Medium" in "1-2. Definition of Terms" shown above, animal cells are preferred, and human cells are more preferred. With respect to the type of cells, the cells are pluripotent stem cells, and pluripotent stem cells like iPS cells and ES cells are particularly preferred.

### (Culture Vessel)

A vessel the inner surface of which is less adhesive to cells is preferred as the culture vessel to be used for culture. Examples of the vessel the inner surface of which is less adhesive to cells include a plate subjected to hydrophilic surface treatment with a biocompatible substance. For example, a Nunclon (TM) Sphera (Thermo Fisher Scientific K.K.) can be used as the culture vessel.

The shape of the culture vessel is not limited, and examples of the culture vessel include dish-like, flask-like, well-like, bag-like, and spinner-flask-like culture vessels.

The capacity of the culture vessel to be used is not limited, and an appropriate capacity can be selected; however, it is preferable that the lower limit of the area of the bottom of a part to contain medium in plan view be 0.32 cm² or larger, 0.65 cm² or larger, 1.9 cm² or larger, 3.0 cm² or larger, 3.5 cm² or larger, 9.0 cm² or larger, or 9.6 cm² or larger, and the upper limit thereof be 1000 cm² or smaller, 500 cm² or smaller, 300 cm² or smaller, 150 cm² or smaller, 75 cm² or smaller, 55 cm² or smaller, 25 cm² or smaller, 21 cm² or smaller, 9.6 cm² or smaller, or 3.5 cm² or smaller.

### (Medium)

The volume of medium or culture solution can be appropriately adjusted for the culture vessel to be used. If a 12-well plate (area of well bottom per well in plan view: 3.5 cm²) is used, for example, the volume per well can be 0.5 mL or more, 1.5 mL or less, and preferably approximately 1.3 mL. If a 6-well plate (area of well bottom per well in plan view: 9.6 cm²) is used, the lower limit of the volume per well can be 1.5 mL or more, 2 mL or more, or 3 mL or more, and the upper limit thereof can be 6.0 mL or less, 5 mL or less, or 4 mL or less. If a 125-mL Erlenmeyer flask (an Erlenmeyer flask having a capacity of 125 mL) is used, the lower limit of the volume per vessel can be 10 mL or more, 15 mL or more, 20 mL or more, 25 mL or more, or 30 mL or more, and the upper limit thereof can be 50 mL or less, 45 mL or less, or 40 mL or less. If an Erlenmeyer flask having a capacity of 500 mL is used, the lower limit of the volume per vessel can be 100 mL or more, 105 mL or more, 110 mL or more, 115 mL or more, or 120 mL or more, and the upper limit thereof can be 150 mL or less, 145 mL or less, 140 mL or less, 135 mL or less, 130 mL or less, or 125 mL or less. If an Erlenmeyer flask having a capacity of 1000 mL is used, the lower limit of the volume per vessel can be 250 mL or more, 260 mL or more, 270 mL or more, 280 mL or more, or 290 mL or more, and the upper limit thereof can be 350 mL or less, 340 mL or less, 330 mL or less, 320 mL or less, or 310 mL or less. If a disposable culture bag, for example, having a capacity of 2 L is used, the lower limit of the volume per bag can be 100 mL or more, 200 mL or more, 300 mL or more, 400 mL or more, 500 mL or more, 600 mL or more, 700 mL or more, 800 mL or more, 900 mL or more, or 1000 mL or more, and the upper limit thereof can be 2000 mL or less, 1900 mL or less, 1800 mL or less, 1700 mL or less, 1600 mL or less, 1500 mL or less, 1400 mL or less, 1300 mL or less, 1200 mL or less, or 1100 mL or less. If a disposable culture bag having a capacity of 10 L is used, the lower limit of the volume per bag can be 500 mL or more, 1 L or more, 2 L or more, 3 L or more, 4 L or more, or 5 L or more, and the upper limit thereof can be 10 L or less, 9 L or less, 8 L or less, 7 L or less, or 6 L or less.

### (Seeding Density)

The density of cells to be seeded (seeding density) in new medium in suspension culture can be appropriately adjusted in view of culture time, the condition of cells after culture, and the number of cells needed after culture. Typically, the seeding density can be in such a range that the lower limit is 0.01 × 10⁵ cells/mL or more, 0.1 × 10⁵ cells/mL or more, 1 × 10⁵ cells/mL or more, or 2 × 10⁵ cells/mL or more, and the upper limit is 20 × 10⁵ cells/mL or less or 10 × 10⁵ cells/mL or less, though the lower limit and upper limit are not limited thereto.

### (Culture Conditions)

There is no limitation to the culture conditions including culture temperature, time, and CO₂ concentration. Culture can be performed with any of conventional methods in the art. For the culture temperature, for example, the lower limit can be 20°C or higher or 35°C or higher, and the upper limit can be 45°C or lower or 40°C or lower, though the culture temperature is preferably 37°C. The culture time can be in such a range that the lower limit is 0.5 hours or longer or 6 hours or longer, and the upper limit is 7 days or shorter, 120 hours or shorter, 96 hours or shorter, 72 hours or shorter, or 48 hours or shorter. For the CO₂ concentration in culture, the lower limit can be 4% or more or 4.5% or more, and the upper limit can be 10% or less or 5.5% or less, though the CO₂ concentration in culture is preferably 5%. Medium exchange can be performed at an appropriate frequency. The frequency of medium exchange varies among cell types to be cultured, and medium exchange can be performed, for example, one or more times per 5 days, one or more times per 4 days, one or more times per 3 days, one or more times per 2 days, one or more times per day, or two or more times per day, though the frequency is not limited thereto. Medium exchange can be performed in such a manner that cells are collected with the same method as in the collection step, fresh medium is then added, and the cell aggregate is gently dispersed, and then cultured again. The frequency of and method or the like for medium exchange are not limited to the above frequency and method, and an optimum method can be appropriately employed.

The timing of termination of culture and the timing of medium exchange may be determined, for example, on the basis of lactic acid concentration in medium. Lactic acid is produced by cells during culture, and accumulated in medium. Lactic acid produced by cells or lactic acid originally contained in medium is known to damage cells, thereby inhibiting the maintenance of the undifferentiation of pluripotent stem cells to lead to adverse effects on proliferation such as lowered cell proliferative capacity, in particular, after subculture. The inhibitory effect of lactic acid on maintenance of undifferentiation and the lowering effect of lactic acid on cell proliferative capacity can be avoided by determining the timing of termination of culture and/or the timing of medium exchange on the basis of lactic acid concentration in medium.

In particular, the effects of lactic acid can be reduced in suspension culture of pluripotent stem cells in the presence of a protein kinase Cβ (PCKβ) inhibitor and a tankyrase (TNKS) inhibitor. In other words, in suspension culture of pluripotent stem cells in the presence of a protein kinase Cβ (PKCβ) inhibitor and a tankyrase (TNKS) inhibitor, the undifferentiation can be maintained or the cell proliferative capacity can be maintained even under higher lactic acid concentration than in common cell culture. Specifically, culture can be performed in a favorable manner even when the lactic acid concentration in medium reaches 5 mM or more. Moreover, culture can be performed in a favorable manner even when the lactic acid concentration in medium reaches 7 mM or more, 9 mM or more, 10 mM or more, 11 mM or more, or 12 mM or more.

It is preferable that the lactic acid concentration in medium be up to 15 mM In particular, it is preferable that the lactic acid concentration in medium be 14 mM or less, 13 mM or less, 12 mM or less, or 10 mM or less. With the lactic acid concentration in medium being within the range, significant lowering of the pH of medium can be prevented, and the above-described effects on cells can be avoided.

As described above, in suspension culture of pluripotent stem cells in the presence of a protein kinase Cβ (PKCβ) inhibitor and a tankyrase (TNKS) inhibitor, the timing of termination of culture and the timing of medium exchange can be set to a timing when the lactic acid concentration in medium reaches, for example, 15 mM, 14 mM or less, 13 mM or less, 12 mM or less, or 10 mM or less. In another embodiment, the timing of termination of culture and the timing of medium exchange may be set to a time at which the lactic acid concentration in medium is lower than the range.

### (Culture Method)

The state of flowing of medium in culture is not limited. Both static culture and flow culture are applicable, but flow culture is preferred.

"Static culture" refers to culture in which medium is left to stand in a culture vessel. The static culture is typically employed for adherent culture.

"Flow culture" refers to culture under conditions allowing medium to flow. Methods of flowing medium to promote the aggregation of cells are preferred for flow culture. Examples of such culture methods include a rotational culture method, a rocking culture method, stirring culture, and combinations of them.

The "rotational culture method" (including a shaking culture method) refers to a method of culturing under conditions allowing medium to flow so that cells can gather to one point by stress (centrifugal force, centripetal force) due to a rotational flow. Specifically, rotational culture is performed by rotating a culture vessel containing medium with cells along a closed trajectory such as a circle, an ellipse, a flattened circle, and a flattened ellipse in a generally horizontal plane.

The rotational speed is not limited, and the lower limit can be 1 rpm or higher, 10 rpm or higher, 50 rpm or higher, 60 rpm or higher, 70 rpm or higher, 80 rpm or higher, 83 rpm or higher, 85 rpm or higher, or 90 rpm or higher. The upper limit can be 200 rpm or lower, 150 rpm or lower, 120 rpm or lower, 115 rpm or lower, 110 rpm or lower, 105 rpm or lower, 100 rpm or lower, 95 rpm or lower, or 90 rpm or lower. The amplitude of a shaker to be used for rotational culture is not limited, and the lower limit can be, for example, 1 mm or larger, 10 mm or larger, 20 mm or larger, or 25 mm or larger. The upper limit can be, for example, 200 mm or smaller, 100 mm or smaller, 50 mm or smaller, 30 mm or smaller, or 25 mm or smaller. Likewise, the rotational radius in rotational culture is not limited, and preferably set in such a manner that the amplitude falls within the above range. The lower limit of the rotational radius is, for example, 5 mm or larger or 10 mm or larger, and the upper limit thereof can be, for example, 100 mm or smaller or 50 mm or smaller. In particular, use of rotation conditions satisfying the above ranges is preferred, for example, in a method for producing a cell aggregate, described later, because a cell aggregate of proper size can be readily produced.

The "rocking culture method" refers to a method of culturing under conditions to provide medium with a rocking flow through linear reciprocating motion such as rocking stirring. Specifically, rocking culture is performed by rocking a culture vessel containing medium with cells in a plane generally perpendicular to the horizontal plane. The rocking speed is not limited, and rocking can be performed in such a manner that the lower limit as one reciprocating motion is regarded as one cycle is, for example, 2 cycles or more, 4 cycles or more, 6 cycles or more, 8 cycles or more, or 10 cycles or more per minute, and the upper limit is 15 cycles or less, 20 cycles or less, 25 cycles or less, or 50 cycles or less per minute. In rocking, it is preferable to slightly incline the culture vessel to the vertical plane, in other word, provide the culture vessel with a guide angle. The rocking angle is not limited, and the lower limit can be, for example, 0.1° or larger, 2° or larger, 4° or larger, 6° or larger, or 8° or larger, and the upper limit can be 20° or smaller, 18° or smaller, 15° or smaller, 12° or smaller, or 10° or smaller. In particular, use of rocking conditions satisfying the above ranges is preferred, for example, in a method for producing a cell aggregate, described later, because a cell aggregate of proper size can be readily produced.

Furthermore, culture can be performed with stirring by motion as a combination of the rotation and rocking.

The "stirring culture method" refers to a method of culturing under conditions involving stirring medium in a culture vessel by using a stirring device such as a stirrer bar and a stirring blade with the culture vessel left to stand. For example, stirring culture can be achieved by using a spinner-flask-like culture vessel equipped with a stirring blade. Such culture vessels are commercially available, and they may be used. In the case of a commercially available spinner-flask-like culture vessel, a cell culture composition in an amount recommended by the manufacturer can be used in a favorable manner. The stirring speed of the stirring device is not limited, and the lower limit can be 1 rpm or higher, 10 rpm or higher, 30 rpm or higher, 50 rpm or higher, or 70 rpm or higher, 90 rpm or higher, 110 rpm or higher, or 130 rpm or higher. The upper limit can be 200 rpm or lower or 150 rpm or lower.

In the "stirring culture method", it is preferable to control the shear stress applied to cells during culture. Generally, most animal cells including pluripotent stem cells are more susceptible to physical stress than other cells. Accordingly, if the shear stress applied to cells in stirring culture is excessively high, the cells may be physically damaged to result in lowered proliferative capacity, or, for pluripotent stem cells, failure in maintaining the undifferentiation.

The shear stress applied to cells in stirring culture is not limited, but depends, for example, on the blade tip speed. The blade tip speed is the circumferential speed of the tip of the stirring blade, and determined by blade diameter [m] × pi × rotational frequency [rps] = blade tip speed [m/s]. If the tip shape of the stirring blade gives a plurality of blade diameters, the largest length can be employed.

In particular, the effects of applied shear stress can be reduced in suspension culture of pluripotent stem cells in the presence of a protein kinase Cβ (PKCβ) inhibitor and a tankyrase (TNKS) inhibitor. In other words, in suspension culture of pluripotent stem cells in the presence of a protein kinase Cβ (PKCβ) inhibitor and a tankyrase (TNKS) inhibitor, the undifferentiation can be maintained or the cell proliferative capacity can be maintained even under conditions involving application of higher shear stress than in common cell culture. Specifically, stirring culture can be performed with the undifferentiation of pluripotent stem cells maintained even when the blade tip speed is a very high value of 0.23 m/s or higher.

The blade tip speed is preferably 0.05 m/s or higher, preferably 0.08 m/s or higher, preferably 0.10 m/s or higher, preferably 0.13 m/s or higher, preferably 0.17 m/s or higher, preferably 0.20 m/s or higher, preferably 0.23 m/s or higher, preferably 0.25 m/s or higher, and preferably 0.30 m/s or higher. With the blade tip speed being within the range, excessive cell-to-cell aggregation can be prevented with the undifferentiation of pluripotent stem cells maintained.

The blade tip speed is preferably 1.37 m/s or lower, preferably 1.00 m/s or lower, preferably 0.84 m/s or lower, preferably 0.50 m/s or lower, preferably 0.42 m/s or lower, preferably 0.34 m/s or lower, and preferably 0.30 m/s or lower. With the blade tip speed being within the range, the state of flowing of medium in the culture system can be stabilized with the undifferentiation of pluripotent stem cells maintained.

To what degree the number of cells is increased and what state cells are to be brought into in the present step can be appropriately determined according to the type of cells to be cultured, the purpose of cell aggregation, the type of medium, and the culture conditions.

The size of each of individual cell aggregates to be produced by the suspension culture method of the present aspect is not limited, and the lower limit of the size in maximum width in an image observed through a microscope can be 30 µm or larger, 40 µm or larger, 50 µm or larger, 60 µm or larger, 70 µm or larger, 80 µm or larger, 90 µm or larger, or 100 µm or larger. The upper limit can be 1000 µm or smaller, 900 µm or smaller, 800 µm or smaller, 700 µm or smaller, 600 µm or smaller, 500 µm or smaller, 400 µm or smaller, or 300 µm or smaller. Cell aggregates sized within the range allow even inner cells therein to be well supplied with oxygen and nutrient components, being preferable as an environment for proliferation of cells.

It is preferable that, in a population of cell aggregates to be produced by the suspension culture method of the present aspect, 30% or more, 40% or more, 50% or more, 60% or more, 70% or more, 75% or more, 80% or more, 85% or more, 90% or more, 95% or more, 98% or more, or 100% of the cell aggregates, each being a weight-based lower limit, be cell aggregates having a size within the above range.

It is preferable that the percentage of viable cells (survival rate) in cells constituting a population of cell aggregates to be produced by the suspension culture method of the present aspect be, for example, 50% or higher, 60% or higher, 70% or higher, 80% or higher, or 90% or higher. Cell aggregates having a survival rate within the range can readily maintain the aggregated state, being in a state preferable for proliferation of cells.

### (Post-Treatment)

After the present step, the culture solution and cells are separated with a conventional method, and the cells are collected. At that time, it is preferable to collect the cells as single cells via detachment or dispersion treatment. Specific methods therefor will be described in detail later for the collection step. The cells collected can be subjected to the next step directly, or after washing with buffer (including PBS buffer), physiological saline, or medium (preferred is medium to be used in the next step or minimal essential medium), as necessary.

### 2-2-2. Suspension Culture Step

The "suspension culture step" is a step of performing suspension culture in medium containing the above-described differentiation inhibitor for pluripotent stem cells or the active ingredients PKCβ inhibitor and TNKS inhibitor.

The cell culture method in the present step is fundamentally equivalent to the culture method described in "2-2-1. Maintenance Culture Step" shown above. Hence, description on matters in common with the method described above for the maintenance culture step is omitted, and only matters characteristic to the present step will be described in detail in the following.

### (Cells)

The cells to be used in the present step are not limited, and preferably cells prepared after the maintenance culture step. With respect to the type of cells, the cells are pluripotent stem cells as described for the maintenance culture step, and pluripotent stem cells like iPS cells and ES cells are particularly preferred. With respect to the state of cells, it is preferable that the cells in seeding in medium be single cells. One cell or a cell population including a plurality of cells (pluripotent stem cell population) may be used as the pluripotent stem cells to be used in the present step. If the pluripotent stem cells are forming a pluripotent stem cell population, the percentage (proportion) of cells expressing a pluripotent stem cell marker (e.g., OCT4, SOX2, Nanog) and/or being positive for a pluripotent stem cell marker in the population is, for example, 90% or higher, 91% or higher, 92% or higher, 93% or higher, 94% or higher, 95% or higher, 96% or higher, 97% or higher, 98% or higher, 99% or higher, or 100% or lower.

### (Medium)

The present step is characterized in that culture is performed in medium containing a PKCβ inhibitor and a TNKS inhibitor, and the medium differs in this respect from medium to be used in the maintenance culture step. The type of the medium is not limited as long as the medium contains a PKCβ inhibitor and a TNKS inhibitor and allows cells to proliferate and/or be maintained.

The concentrations of a PKCβ inhibitor and a TNKS inhibitor contained in the medium in the present step are as described in the section 1-3. Configuration shown above. A PKCβ inhibitor and a TNKS inhibitor may be added in any manner without limitation, as long as the concentrations of a PKCβ inhibitor and a TNKS inhibitor in the medium at the beginning of the present step fall within the above ranges. For example, the medium may be prepared by directly administering one or more PKCβ inhibitors and TNKS inhibitors to the medium so that each concentration in total falls within the corresponding concentration range shown above.

### (Culture Method)

Suspension culture is performed in the present step. Accordingly, flow culture, in which medium is allowed to flow, is preferred as the culture method. Suspension culture of pluripotent stem cells in the medium containing a PKCβ inhibitor and a TNKS inhibitor allows the pluripotent stem cells to maintain the undifferentiated state.

In the present step, some of pluripotent stem cells in the course of culture can be taken out for checking whether the undifferentiated state is maintained. For example, whether the undifferentiated state is maintained can be checked by measuring expression of a pluripotent stem cell marker expressed on pluripotent stem cells taken out during culture. Examples of pluripotent stem cell markers include, as described above, Alkaline Phosphatase, Nanog, OCT4, SOX2, TRA-1-60, c-Myc, KLF4, LIN28, SSEA-4, and SSEA-1. Examples of methods for detecting these pluripotent stem cell markers include, as described above, flow cytometry.

If the positive rate of pluripotent stem cells taken out during culture for a pluripotent stem cell marker is preferably 80% or higher, more preferably 90% or higher, more preferably 91% or higher, more preferably 92% or higher, more preferably 93% or higher, more preferably 94% or higher, more preferably 95% or higher, more preferably 96% or higher, more preferably 97% or higher, more preferably 98% or higher, more preferably 99% or higher, or more preferably 100% or lower, it can be determined that the undifferentiation is maintained.

In the present step, whether the undifferentiated state is maintained can be checked by measuring expression of three germ layer markers (endodermal cell marker, mesodermal cell marker, and ectodermal cell marker) in pluripotent stem cells taken out in the course of culture. Specifically, if all of the positive rates for the endodermal cell marker, mesodermal cell marker, and ectodermal cell marker are preferably 20% or lower, more preferably 10% or lower, more preferably 9% or lower, more preferably 8% or lower, more preferably 7% or lower, more preferably 6% or lower, more preferably 5% or lower, more preferably 4% or lower, more preferably 3% or lower, more preferably 2% or lower, more preferably 1% or lower, or more preferably below the detection limit, it can be determined that the undifferentiation is maintained.

The endodermal cell marker is a gene specific to endodermal cells, and examples thereof include SOX17, FOXA2, CXCR4, AFP, GATA4, and EOMES. Endodermal cells differentiate to form tissue of an organ such as the gastrointestinal tract, the lung, the thyroid, the pancreas, and the liver; cells of secretory glands opening in the gastrointestinal tract; the peritoneum, the pleura, the larynx, eustachian tubes, the trachea, the bronchi, the urinary tract (the bladder, most part of the urethra, part of the ureter), and others.

The mesodermal cell marker is a gene specific to mesodermal cells, and examples thereof include T (BRACHYURY), MESP1, MESP2, FOXF1, HAND1, EVX1, IRX3, CDX2, TBX6, MIXL1, ISL1, SNAI2, FOXC1, and PDGFRα. Mesodermal cells differentiate to form the celom and the mesothelium lining it, muscles, the skeleton, the dermis, connective tissues, the heart, blood vessels (including vascular endothelia), blood (including blood cells), lymphatic vessels, the spleen, the kidney, the ureter, the gonad (testis, uterus, gonad epithelium), and others.

The ectodermal cell marker is a gene specific to ectodermal cells, and examples thereof include FGF5, NESTIN, SOX1, and PAX6. Ectodermal cells differentiate to form the epidermis, the epithelium of the end of the male urethra, hair, nails, skin grands (including mammary glands and sweat glands), sense organs (including the oral cavity, the pharynx, the nose, and the epithelium of the end of the rectum, and salivary glands) the lenses, the peripheral nervous system, and others. A part of the ectoderm invaginates like a groove to form a neural tube in the developmental process, which in term serves as the origin of neurons in the central nervous system including the brain and the spinal cord, and melanocytes.

Expression of those three germ layer markers (endodermal cell marker, mesodermal cell marker, and ectodermal cell marker) can be measured with any detection method in the art. Examples of methods for measuring expression of the three germ layer markers (endodermal cell marker, mesodermal cell marker, and ectodermal cell marker) include, but are not limited to, quantitative real-time PCR analysis, an RNA-Seq method, Northern hybridization, and a hybridization method using a DNA array. In quantitative real-time PCR analysis, the expression level of a marker gene as a target of measurement is converted into a relative expression level to the expression level of an internal standard gene, and the expression level of the marker gene can be evaluated on the basis of the relative expression level. Examples of the internal standard gene can include a glyceraldehyde-3-phosphate dehydrogenase (GAPDH) gene and a β-actin (ACTB) gene.

### 2-2-3. Collection Step

The "collection step", an optional step in the method of the present invention, is a step of collecting cultured cells from the culture solution after the maintenance culture step or the suspension culture step.

In the present specification, the term "collection (of cells)" means obtaining cells by separating culture solution and cells. A method of collecting cells according to a conventional method used in cell culture methods in the art can be used, without limitation.

After the step of suspension culture, cells are present in a suspended state in the culture solution. Accordingly, collection of cells can be achieved by removing liquid components, the supernatant, by leaving to stand or through centrifugation. Alternatively, cells may be collected with a filtration filter, a hollow fiber separation membrane, or the like. If liquid components are removed by leaving to stand, the vessel containing the culture solution is left to stand for about 5 minutes, and the supernatant can be removed with precipitated cells or cell aggregates left unremoved. Centrifugation can be performed with such a centrifugal acceleration and treatment time that cells are not damaged by the centrifugal force. For example, the lower limit of the centrifugal acceleration is not limited as long as cells can be precipitated, and can be, for example, 100 ×g or higher, 300 ×g or higher, 800 ×g or higher, or 1000 ×g or higher. The upper limit can be such a speed that cells are nod damaged or hardly damaged by the centrifugal force, and can be, for example, 1400 ×g or lower, 1500 ×g or lower, or 1600 ×g or lower. The lower limit of the treatment time may be any time that allows cells to be precipitated by the centrifugal acceleration without limitation, and can be, for example, 30 seconds or longer, 1 minute or longer, 3 minutes or longer, or 5 minutes or longer. The upper limit can be such a time that cells are not damaged or hardly damaged by the centrifugal acceleration, and can be, for example, 10 minutes or shorter, 8 minutes or shorter, 6 minutes or shorter, or 30 seconds or shorter. If liquid components are removed through filtration, for example, the culture solution is passed through a nonwoven fabric or a mesh filter to remove the filtrate, and the residual cell aggregates can be collected. If liquid components are removed with a hollow fiber separation membrane, for example, cells can be collected by separating the culture solution and the cells with use of an apparatus including a hollow fiber separation membrane such as a Cell Washing Concentration System (KANEKA CORPORATION).

The cells collected can be washed, as necessary. The washing method is not limited. For example, washing can be performed in the same manner as the washing method described in "Post-Treatment" in the maintenance culture step shown above. For the washing solution, buffer (including PBS buffer), physiological saline, or medium (minimal essential medium is preferred) can be used.

### (Single-Cell Formation)

In the present specification, "single-cell formation" refers to forming single, free cells by dispersing a cell assembly in which a plurality of cells is adhering to each other or aggregating, such as a monolayer piece of cells and a cell aggregate.

In single-cell formation, a detaching agent and/or a chelating agent is used. The detaching agent is not limited, and, for example, trypsin, collagenase, Pronase, hyaluronidase, and elastase are applicable, and commercially available Accutase (R), Accumax (R), TrypLE (TM) Express Enzyme (Life Technologies Japan Ltd.), TrypLE (TM) Select Enzyme (Life Technologies Japan Ltd.), Dispase (R), and the like are also applicable. If trypsin is used for single-cell formation, for example, the lower limit of the concentration in the solution may be any concentration that allows a cell assembly to be dispersed without limitation, and can be, for example, 0.15 vol% or more, 0.18 vol% or more, 0.20 vol% or more, or 0.24 vol% or more. The upper limit of the concentration in the solution may be any concentration such that cells themselves are not affected, for example, not dissolved, without limitation, and can be 0.30 vol% or less, 0.28 vol% or less, or 0.25 vol% or less. Although the treatment time depends on the concentration of trypsin, the lower limit may be any time that allows a cell assembly to be sufficiently dispersed by the action of trypsin without limitation, and can be, for example, 5 minutes or longer, 8 minutes or longer, 10 minutes or longer, 12 minutes or longer, or 15 minutes or longer. The upper limit of the treatment time may be any time such that cells themselves are not affected, for example, not dissolved by the action of trypsin without limitation, and can be, for example, 30 minutes or shorter, 28 minutes or shorter, 25 minutes or shorter, 22 minutes or shorter, 20 minutes or shorter, or 18 minutes or shorter. If a commercially available detaching agent is used, the commercially available detaching agent can be used at a concentration that allows cells to be dispersed into single forms, as shown in the attached protocol. Single-cell formation can be promoted by mild physical treatment after the treatment with a detaching agent and/or a chelating agent. This physical treatment is not limited, and examples thereof include multiple times of pipetting for cells together with solution. In addition, cells may be passed through a strainer or a mesh, as necessary.

The cells subjected to single-cell formation can be collected by removing the supernatant containing the detaching agent by leaving to stand or through centrifugation. The cells collected may be washed, as necessary. The conditions for centrifugation and the washing method can be as described above.

### 3. Method for Producing Cell Aggregate and Cell Aggregate Obtained by The Same Method

### 3-1. Summary

A cell aggregate can be obtained as a product by applying "2. Method for Producing Pluripotent Stem Cell Population" shown above. The present production method allows a cell aggregate to be produced with the undifferentiated state of pluripotent stem cells maintained.

### 3-2. Method for Producing Cell Aggregate

Basic steps in the method of the present aspect for producing a cell aggregate are equivalent to those of "2. Method for Producing Pluripotent Stem Cell Population" shown above. Thus, the method of the present aspect includes: a suspension culture step as an essential step; and a maintenance culture step and a collection step as optional steps. The steps are as described above.

### 3-3. Cell Aggregate

A cell aggregate with an undifferentiated state maintained can be produced by the method of the present aspect for producing a cell aggregate.

The size of each of individual cell aggregates to be produced by the method of the present aspect for producing a cell aggregate is not limited, and the lower limit of the size in maximum width in an image observed through a microscope can be 30 µm or larger, 40 µm or larger, 50 µm or larger, 60 µm or larger, 70 µm or larger, 80 µm or larger, 90 µm or larger, or 100 µm or larger. The upper limit can be 1000 µm or smaller, 900 µm or smaller, 800 µm or smaller, 700 µm or smaller, 600 µm or smaller, 500 µm or smaller, 400 µm or smaller, 300 µm or smaller, or 200 µm or smaller. For reference, a human iPS cell has a dimension of approximately 10 µm. Cell aggregates sized within the range allow even inner cells therein to be well supplied with oxygen and nutrient components, being preferable as an environment for proliferation of cells.

It is preferable that, in a population of cell aggregates to be produced by the method of the present aspect for producing a cell aggregate, 10% or more, 20% or more, 30% or more, 40% or more, 50% or more, 60% or more, 70% or more, 75% or more, 80% or more, 85% or more, 90% or more, 95% or more, 98% or more, or 100% of the cell aggregates, each being a weight-based lower limit, be cell aggregates having a size within the above range. Populations of cell aggregates including 20% or more of cell aggregates having a size within the above range allow even inner cells in individual cell aggregates to be well supplied with oxygen and nutrient components, being preferable as an environment for proliferation of cells.

It is preferable that the percentage of viable cells (survival rate) in cells constituting a population of cell aggregates to be produced by the method of the present aspect for producing a cell aggregate be, for example, 50% or higher, 60% or higher, 70% or higher, 80% or higher, or 90% or higher. Cell aggregates having a survival rate within the range can readily maintain the aggregated state, being in a state preferable for proliferation of cells.

If cells constituting a cell aggregate are pluripotent stem cells, the undifferentiation of the pluripotent stem cells constituting the cell aggregate may be determined from the percentage of cells expressing a pluripotent stem cell marker and/or being positive for a pluripotent stem cell marker. For example, the positive rate for a pluripotent stem cell marker can be preferably 80% or higher, more preferably 90% or higher, more preferably 91% or higher, more preferably 92% or higher, more preferably 93% or higher, more preferably 94% or higher, more preferably 95% or higher, more preferably 96% or higher, more preferably 97% or higher, more preferably 98% or higher, more preferably 99% or higher, and more preferably 100% or lower. Cell aggregates having a positive rate within the range are composed of pluripotent stem cell populations with the undifferentiated state maintained, and can be said to be highly homogeneous cell populations.

Pluripotent stem cell markers can be detected with any detection method in the art. Examples of methods for detecting cell markers include, but are not limited to, flow cytometry. In the case that a fluorescence-labeled antibody is used as a detection reagent in flow cytometry, if a cell emitting more intense fluorescence than a negative control (isotype control) is detected, the cell is determined as "positive" for the marker. The proportion of cells positive for a fluorescence-labeled antibody as analyzed by flow cytometry is occasionally referred to as the "positive rate". Any antibody known in the art can be used for such fluorescence-labeled antibodies, and examples thereof include, but are not limited to, antibodies labeled with fluorescein isothiocyanate (FITC), phycoerythrin (PE), or allophycocyanin (APC).

In the present invention, cells constituting a cell aggregate may be composed of a single type or two or more types of cells. For example, cell aggregates composed only of iPS cells are acceptable, and cell aggregates composed of iPS cells and ES cells are also acceptable.

### 3-4. Advantageous Effects

The method of the present aspect for producing a cell aggregate allows a cell aggregate to be produced with the undifferentiated state of pluripotent stem cells maintained.

### 4. Method for Producing Pluripotent Stem Cell Cluster

### 4-1. Summary

A pluripotent stem cell aggregate can be produced with "2. Method for Producing Pluripotent Stem Cell Population" shown above. The present production method allows a cell aggregate to be produced with the undifferentiated state of pluripotent stem cells maintained.

### 4-2. Method for Producing Pluripotent Stem Cell Cluster

Basic steps in the method of the present aspect for producing a pluripotent stem cell cluster are equivalent to those of "2. Method for Producing Pluripotent Stem Cell Population" shown above. Thus, the method of the present aspect includes: a step of performing suspension culture in medium containing a PKCβ inhibitor and a TNKS inhibitor (first step) as an essential step; and a maintenance culture step and a collection step as optional steps. In principle, the steps are as described in "2. Method for Producing Pluripotent Stem Cell Population" shown above.

The culture time in the first step is preferably 0.5 hours or longer, more preferably 12 hours or longer, and preferably 7 days or shorter, 6 days or shorter, 5 days or shorter, 4 days or shorter, more preferably 72 hours or shorter, more preferably 48 hours or shorter, most preferably 24 hours or shorter. The culture time in the maintenance culture step is preferably 0.5 hours or longer, more preferably 12 hours or longer, 24 hours or longer, 48 hours or longer, and preferably 7 days or shorter, 6 days or shorter, 5 days or shorter, more preferably 4 days or shorter, most preferably 72 hours or shorter.

Medium exchange can be performed during the first step, between the first step and the maintenance culture step, and/or during the maintenance culture step at an appropriate frequency. In particular, medium exchange is performed between the first step and the maintenance culture step. The frequency of medium exchange varies among cell types to be cultured, and medium exchange can be performed, for example, one or more times per 5 days, one or more times per 4 days, one or more times per 3 days, one or more times per 2 days, one or more times per day, or two or more times per day, though the frequency is not limited thereto. The frequency and method or the like of medium exchange are not limited to the above frequency and method, and an optimum method can be employed as appropriate.

### 4-3. Pluripotent Stem Cell Aggregate

The method of the present aspect for producing a pluripotent stem cell cluster allows a pluripotent stem cell aggregate of proper size to be produced with the undifferentiated state of pluripotent stem cells maintained. For example, the percentage (proportion) of cells expressing a pluripotent stem cell marker (e.g., OCT4, SOX2, NANOG) and/or being positive for a pluripotent stem cell marker in a pluripotent stem cell population obtained with the method of the present aspect for culturing a pluripotent stem cell population is, for example, 90% or higher, 91% or higher, 92% or higher, 93% or higher, 94% or higher, 95% or higher, 96% or higher, 97% or higher, 98% or higher, 99% or higher, or 100% or lower.

### 4-4. Advantageous Effects

The method of the present aspect for producing a cell aggregate allows a cell aggregate to be produced with the undifferentiated state of pluripotent stem cells maintained.

### 5. Medium for Suspension Culture

### 5-1. Summary

The present aspect is medium for suspension culture of cells. With the medium of the present aspect for suspension culture, the undifferentiated state of pluripotent stem cells in the medium can be maintained.

### 5-2. Configuration

The medium of the present aspect for suspension culture of cells is composed of cell proliferation medium for maintaining the undifferentiated state of pluripotent stem cells in suspension culture, the medium at least containing the differentiation inhibitor described in "1. Differentiation inhibitor for Pluripotent Stem Cells" shown above or the active ingredients PKCβ inhibitor and TNKS inhibitor.

The composition of the medium of the present aspect for suspension culture has been described in detail in the subsection "Culture and Medium" in "1-2. Definition of Terms" of "1. Differentiation inhibitor for Pluripotent Stem Cells" shown above.

The configurations of the differentiation inhibitor and the active ingredients PKCβ inhibitor and TNKS inhibitor, and the concentrations thereof comprised in the differentiation inhibitor have been described in detail in "1.3 Configuration" of "1. Differentiation inhibitor for Pluripotent Stem Cells" shown above.

Although the medium for suspension culture is used as liquid medium at least in cell culture, the medium for suspension culture may be powdered or formulated into a solid state for storage. In this case, the solid can be dissolved in sterile water or the like to dilute to an appropriate concentration before use as medium.

The medium for suspension culture can be cryopreserved until use, and can be thawed in use.

### 5-3. Advantageous Effects

Suspension culture of cells with the medium of the present aspect for suspension culture allows pluripotent stem cells to maintain the undifferentiated state.

### 6. Differentiation Suppression Kit for Pluripotent Stem Cells

### 6-1. Summary

The kit comprising a differentiation inhibitor for pluripotent stem cells according to the present invention is a kit for use in culture of pluripotent stem cells, wherein the kit comprises a differentiation inhibitor for pluripotent stem cells, and the differentiation inhibitor contains a PKCβ inhibitor and a TNKS inhibitor. With the present differentiation suppression kit for pluripotent stem cells, pluripotent stem cells can be proliferated through suspension culture with the undifferentiated state of the pluripotent stem cells maintained.

### 6-2. Configuration

The differentiation suppression kit for pluripotent stem cells includes: a differentiation inhibitor for pluripotent stem cells, as an essential component, the differentiation inhibitor containing a PKCβ inhibitor and a TNKS inhibitor; and medium and/or a protocol as optional components.

The protocol describes how to use the present differentiation suppression kit for pluripotent stem cells. Specifically, the loadings of the PKCβ inhibitor and TNKS inhibitor included in the differentiation suppression kit for pluripotent stem cells to medium, the loadings of other components, a favorable suspension culture method for maintaining undifferentiation, and so on.

### Examples

Hereinafter, the differentiation inhibitor for pluripotent stem cells and method for producing a pluripotent stem cell population according to the present invention will be described in more detail with reference to Examples; however, the technical scope of the present invention is not limited to Examples in the following.

### <Reference Example 1: Adherent Culture of Human iPS Cell WTC-11 Strain>

Human iPS cell WTC-11 strain (Coriell Institute) was seeded on a cell culture dish coated with 0.25 µg/cm² of iMatrix-511 Silk (Nippi, Incorporated), and subjected to adherent culture under an atmosphere at 37°C and 5% CO₂. The medium used was StemFit (R) AK02N (Ajinomoto Co., Inc.), and medium exchange was performed every day. Only in seeding the cells, a ROCK inhibitor (Y-27632 (FUJIFILM Wako Pure Chemical Corporation)) was added to the medium to give a final concentration of 10 µM.

### <Comparative Example 1: Suspension Culture of Human iPS Cell WTC-11 Strain>

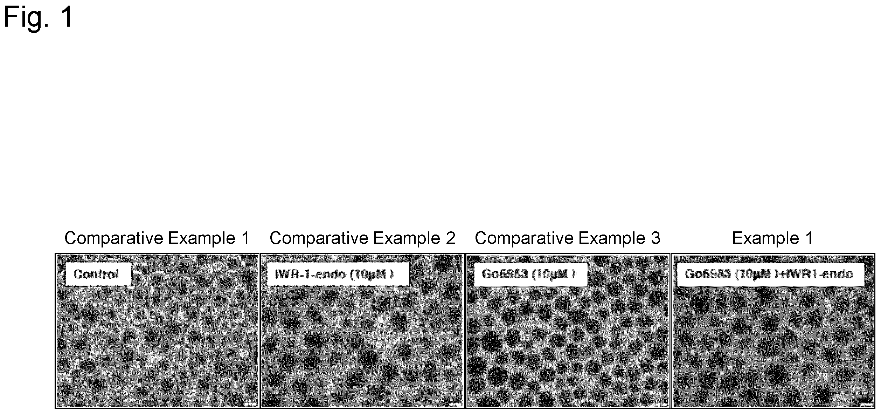

Human iPS cell WTC-11 strain subjected to adherent culture with the procedure in Reference Example 1 was washed with Dulbecco's PBS (NACALAI TESQUE, INC.), and then treated with 0.5 mM EDTA solution in Dulbecco's PBS (NACALAI TESQUE, INC.) for 5 minutes. After the EDTA solution was sucked up, StemFit (R) AK02N medium was added to the dish, cells were detached with a cell scraper, and then dispersed into single cells by pipetting. The cells were suspended in StemFit (R) AK02N medium containing Y-27632 (FUJIFILM Wako Pure Chemical Corporation) with a final concentration of 10 µM, and some of the cells were stained with trypan blue and the number of viable cells was counted. StemFit (R) AK02N medium containing Y-27632 with a final concentration of 10 µM was applied to the cell suspension to prepare a cell suspension containing 1 × 10⁵ cells per 1 mL. Into a 6-well plate for suspension culture (Sumitomo Bakelite Co., Ltd.), 4 mL of the cell suspension was seeded per well. On a rotary shaker (Optima Co., Ltd.), the plate into which the cells had been seeded was rotated along a circle having a rotation width (diameter) of 25 mm in the horizontal plane at a speed of 90 rpm, and suspension culture was performed under an environment at 37°C and 5% CO₂. Subculture was performed on day 5 of culture as the day on which the cells were seeded was defined as day 0 of culture, and suspension culture was performed until day 10 of culture. Medium exchange was performed on day 2, 4, 7, and 9 of culture. Each medium exchange was performed in such a manner that only the culture supernatant in a volume of 3 mL was removed from each well by suction, and 3 mL of StemFit (R) AK02N medium containing no Y-27632 was added to each well. On day 5 of culture, a cell aggregate and culture supernatant were collected from each well into a centrifuge tube, left to stand for about 5 minutes to precipitate the cell aggregate, and the culture supernatant was removed. After washing with Dulbecco's PBS, 1 mL of Accutase was added to each cell aggregate for treatment at 37°C for 10 minutes, and the cell aggregate was dispersed into single cells by pipetting. The cells were suspended in StemFit (R) AK02N medium containing Y-27632 with a final concentration of 10 µM, and some of the cells were stained with trypan blue and the number of viable cells was counted. StemFit (R) AK02N medium containing Y-27632 with a final concentration of 10 µM was applied to the cell suspension to prepare a cell suspension containing 1 × 10⁵ cells per 1 mL, and the cells were seeded in the same manner as on day 0 of culture to continue suspension culture. On day 10 of culture, phase-contrast images of cell aggregates were acquired by using a phase-contrast microscope. Figure 1 shows phase-contrast images of cell aggregates. The formation of cell aggregates was confirmed from Figure 1.

### <Comparative Example 2: Addition of TNKS Inhibitor to Suspension Culture of Human iPS Cell WTC-11 Strain>

Suspension culture was carried out with the same procedure as in Comparative Example 1, except that medium for seeding and medium for medium exchange in suspension culture were prepared to allow them to contain the TNKS inhibitor IWR-1-endo (FUJIFILM Wako Pure Chemical Corporation) with a final concentration of 10 µM.

### <Comparative Example 3: Addition of PKCβ Inhibitor to Suspension Culture of Human iPS cell WTC-11 Strain>

Suspension culture was carried out with the same procedure as in Comparative Example 1, except that medium for seeding and medium for medium exchange in suspension culture were prepared to allow them to contain the PKCβ inhibitor Go6983 (FUJIFILM Wako Pure Chemical Corporation) with a final concentration of 10 µM.

### <Example 1: Addition of TNKS Inhibitor and PKCβ Inhibitor to Suspension Culture of Human iPS Cell WTC-11 Strain>

Suspension culture was carried out with the same procedure as in Comparative Example 1, except that medium for seeding and medium for medium exchange in suspension culture were prepared to allow them to contain IWR-1-endo and Go6983 each with a final concentration of 10 µM.

### <Example 2: Quantitative Real-Time PCR Analysis>

Quantitative real-time PCR analysis was performed with a procedure shown in the following. The cells on day 5 of culture in Reference Example 1, and the cells on day 10 of culture in Comparative Examples 1 and 2 and Example 1 were washed with Dulbecco's PBS, and total RNA was purified by using a Monarch Total RNA Miniprep Kit (New England Biolabs Japan Inc.). The purification method was according to a usage manual attached to the kit. Total RNA in an amount of 1000 ng was separated with a nano spectrophotometer. To the RNA separated, 1 µL of ReverTra Ace (R) (TOYOBO CO., LTD.), 4 µL of 5× RT buffer (TOYOBO CO., LTD.), 2 µL of 10 mM dNTPs Mixture (TOYOBO CO., LTD.), 1 µL of RNase inhibitor (10 units/ µL) (TOYOBO CO., LTD.), 1 µL of Random primer (25 pmol/µL) (TOYOBO CO., LTD.), and RNase Free dH₂O were added to prepare 20 µL of solution, and cDNA synthesis was performed by using a SimpliAmp Thermal Cycler (Thermo Fisher Scientific). The reaction conditions for the cDNA synthesis were such that reaction at 30°C for 10 minutes was followed by consecutive reactions of reaction at 42°C for 60 minutes and reaction at 99°C for 5 minutes, followed by cooling to 4°C. The synthesized cDNA solution was 5-fold diluted with sterile water. To a reaction plate, 0.5 µL/well of the diluted cDNA solution, 3.98 µL/well of sterile water, 5 µL/well of THUNDERBIRD (R) Probe qPCR Mix (TOYOBO CO., LTD.), 0.5 µL/well of Taqman (R) probes, and 0.02 µL/well of 50X ROX reference dye (TOYOBO CO., LTD.) were added and mixed together. The Taqman probes used were those for GAPDH, OCT4, T, SOX17, and PAX6. Quantitative real-time PCR analysis was carried out by using a TaqMan (R) QuantStudio3 real-time PCR system (Thermo Fisher Scientific). Table 1 shows the reaction conditions.

**[Table 1]**

| Step | Temperature | Time | Number of cycles |
|---|---|---|---|
| 1 | 94°C | 20 sec | - |
| 2 | 95°C | 1 sec | 40 cycle |
| 3 | 60°C | 30 sec | |

The Assay IDs of the Taqman (R) probes used for the quantitative real-time PCR analysis are shown in the following.
GAPDH: Hs02786624_g1
OCT4: Hs01088114_m1
T: Hs00610080_m1
SOX17: Hs00751752_s1
PAX6: Hs04260367_gH

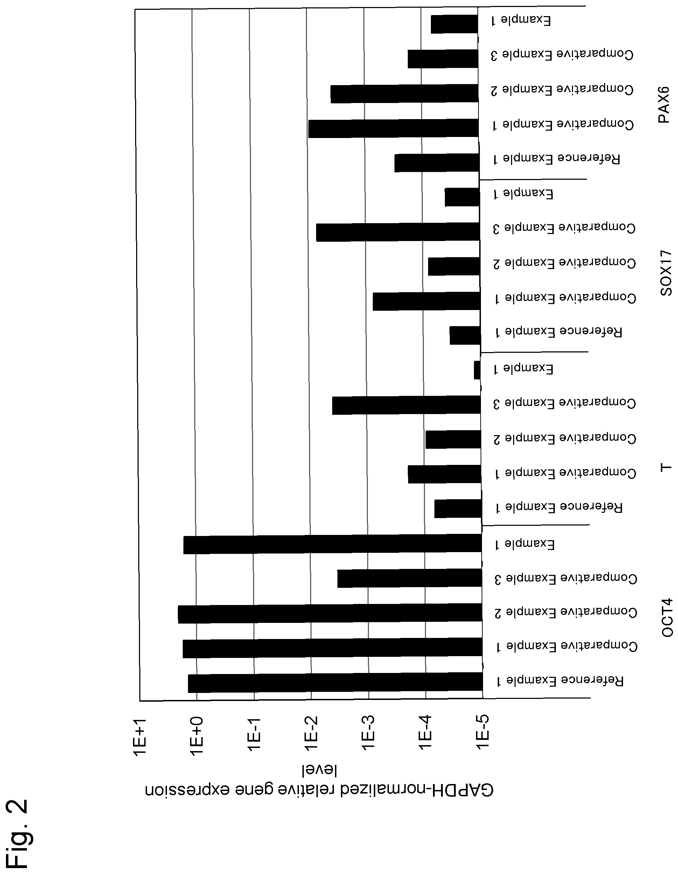

Table 2 and Figure 2 show the results of measurement of gene expression.

**[Table 2]**

| | ACTB-normalized relative gene expression level (2^{-ΔCt}) | | | |
|---|---|---|---|---|
| | OCT4 | T | SOX17 | PAX6 |
| Reference Example 1 | 1.40 | 6.53 × 10⁻⁵ | 3.36 × 10⁻⁵ | 2.97 × 10⁻⁴ |
| Comparative Example 1 | 1.71 | 1.87 × 10⁻⁴ | 7.40 × 10⁻⁴ | 9.35 × 10⁻³ |
| Comparative Example 2 | 2.05 | 9.16 × 10⁻⁵ | 7.86 × 10⁻⁵ | 3.76 × 10⁻³ |
| Comparative Example 3 | 3.30 × 10⁻³ | 3.89 × 10⁻³ | 7.06 × 10⁻³ | 1.68 × 10⁻⁴ |
| Example 1 | 1.62 | 1.28 × 10⁻⁵ | 3.94 × 10⁻⁵ | 6.49 × 10⁻⁵ |

As shown in Table 2 and Figure 2, the expression levels of T (mesoderm marker gene), SOX17 (endoderm marker gene), and PAX6 (ectoderm marker gene) were low and the undifferentiated state of pluripotent stem cells was maintained for Reference Example 1, in which adherent culture was performed.

For Comparative Example 1, in which suspension culture was performed, by contrast, the expression levels of T, SOX17, and PAX6 were higher than those for Reference Example 1. These results suggested that, in the suspension culture, spontaneous differentiation into mesoderm, endoderm, and ectoderm occurred and maintenance of the undifferentiated state of pluripotent stem cells failed. For Comparative Example 2, in which suspension culture was performed with addition of a TNKS inhibitor, reduced expression levels were achieved for T and SOX17 to a degree comparable to that for Reference Example 1, but the expression level of PAX6 was higher than that for Reference Example 1. For Comparative Example 3, in which suspension culture was performed with addition of a PKCβ inhibitor, a reduced expression level was achieved for PAX6 to a degree comparable to that for Reference Example 1, but the expression levels of T and SOX17 were higher than those for Comparative Example 1. For Example 1, in which a TNKS inhibitor and a PKCβ inhibitor were both added, the expression levels of T, SOX17, and PAX6 were all comparable to those for Reference Example 1.

These results demonstrated that the TNKS inhibitor is capable of effectively suppressing spontaneous differentiation into mesoderm and endoderm, but the suppressive effect on ectodermal differentiation is weak; and that the PKCβ inhibitor is capable of suppressing spontaneous differentiation into ectoderm, but disadvantageously promotes mesodermal and endodermal differentiation. On the other hand, it was revealed that suspension culture with addition of both the TNKS inhibitor and the PKCβ inhibitor can suppress spontaneous differentiation into all of mesoderm, endoderm, and ectoderm.

In summary, it was revealed that addition of only one of the TNKS inhibitor and the PKCβ inhibitor is insufficient for effectively suppressing the spontaneous differentiation of pluripotent stem cells into mesoderm, endoderm, and ectoderm and maintaining the undifferentiated state of pluripotent stem cells, and, instead, addition of both the TNKS inhibitor and the PKCβ inhibitor enables suppression of the spontaneous differentiation into all of mesoderm, endoderm, and ectoderm, leading to successful maintenance of the undifferentiated state of pluripotent stem cells.

### <Reference Example 2: Adherent Culture of Human iPS Cell 201B7 Strain>

Human iPS cell 201B7 strain (Center for iPS Cell Research and Application, Kyoto University) was seeded on a cell culture dish coated with 0.5 µg/cm² of Vitronectin (VTN-N) Recombinant Human Protein, Truncated (Thermo Fisher Scientific), and subjected to adherent culture under an atmosphere at 37°C and 5% CO₂. The medium used was StemFit (R) AK02N (Ajinomoto Co., Inc.), and medium exchange was performed every day. Only in seeding the cells, Y-27632 (FUJIFILM Wako Pure Chemical Corporation) was added to the medium to give a final concentration of 10 µM.

### <Comparative Example 4: Suspension Culture of Human iPS Cell 201B7 Strain>

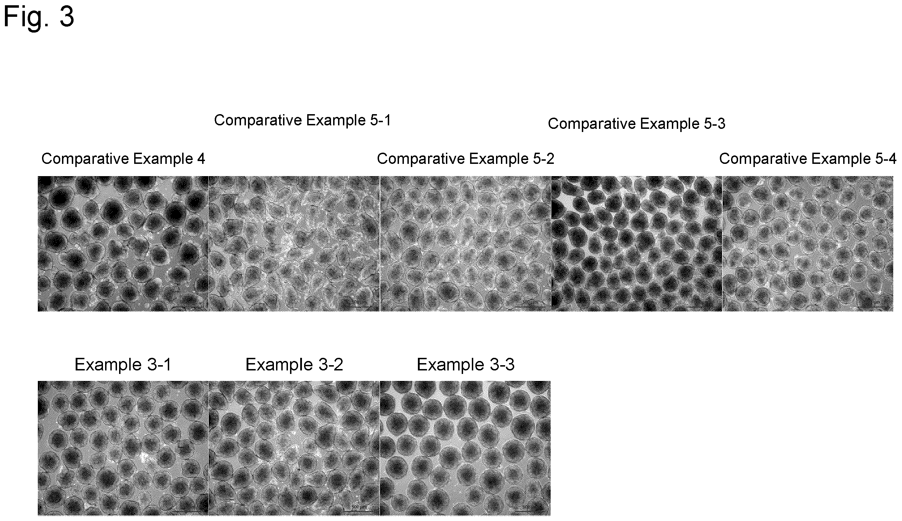

Human iPS cell 201B7 strain subjected to adherent culture with the same procedure as in Reference Example 2 was treated with Accutase (Innovative Cell Technologies, Inc.) for 5 minutes to detach from the culture surface, and dispersed into single cells by pipetting. The cells were suspended in StemFit (R) AK02N medium containing Y-27632 with a final concentration of 10 µM, and some of the cells were stained with trypan blue and the number of viable cells was counted. StemFit (R) AK02N medium containing Y-27632 with a final concentration of 10 µM was applied to the cell suspension to prepare a cell suspension containing 2 × 10⁵ cells per 1 mL. Into a 6-well plate for suspension culture, 4 mL of the cell suspension was seeded per well. On a rotary shaker, the plate into which the cells had been seeded was rotated along a circle having a rotation width (diameter) of 25 mm in the horizontal plane at a speed of 83 rpm, and suspension culture was performed under an environment at 37°C and 5% CO₂. Subculture was performed on day 4 of culture as the day on which the cells were seeded was defined as day 0 of culture, and suspension culture was performed until day 8 of culture. Medium exchange was performed every day. Each medium exchange was performed in such a manner that the whole volume of medium containing a cell aggregate was collected into a centrifuge tube, left to stand for about 5 minutes to precipitate the cell aggregate, the culture supernatant was then removed, and the resultant was gently resuspended in StemFit (R) AK02N medium, which was returned into the original well. In medium exchange, Y-27632 was added to the medium to give a final concentration of 5 µM on day 1 and 5 of culture, and Y-27632 was added to the medium to give a final concentration of 2 µM on day 2 and 6 of culture. On day 4 of culture, each cell aggregate and culture supernatant were collected into a centrifuge tube, left to stand for about 5 minutes to precipitate the cell aggregate, and the culture supernatant was removed. After 1 mL of Accutase was added to each cell aggregate for treatment at 37°C for 10 minutes, the cell aggregate was dispersed into single cells by pipetting. The cells were suspended in StemFit (R) AK02N medium containing Y-27632 with a final concentration of 10 µM, and some of the cells were stained with trypan blue and the number of viable cells was counted. StemFit (R) AK02N medium containing Y-27632 with a final concentration of 10 µM was applied to the cell suspension to prepare a cell suspension containing 2 × 10⁵ cells per 1 mL, and the cells were seeded in the same manner as on day 0 of culture to continue suspension culture. On day 8 of culture, phase-contrast images of cell aggregates were acquired by using a phase-contrast microscope. Figure 3 shows phase-contrast images of cell aggregates. The formation of cell aggregates was confirmed from

Figure 3.

### <Comparative Example 5: Addition of TNKS Inhibitor and PKC Inhibitor to Suspension Culture of Human iPS Cell 201B7 Strain>

To check whether PKC inhibitors that inhibit PKC isozymes other than PKCβ have differentiation-suppressing effect, suspension culture was carried out in the following manner.

Suspension culture was carried out with the same procedure as in Comparative Example 4, except that a combination of a TNKS inhibitor and a PKC inhibitor (that inhibits a PKC isozyme other than PKCβ) shown in Table 3 was added to medium for seeding and medium for medium exchange in suspension culture.

**[Table 3]**

| | TNKS inhibitor | PKC inhibitor |
|---|---|---|
| | Name of reagent/final concentration | Name of reagent/final concentration |
| Comparative Example 5-1 | IWR -1-endo/ 10 µM (FUJIFILM Wako Pure Chemical Corporation) | Staurosporine/1nM (PromoCell GmbH) |
| Comparative Example 5-2 | IWR -1-endo/ 10 µM (FUJIFILM Wako Pure Chemical Corporation) | ZIP/1 µM (abcam) |
| Comparative Example 5-3 | IWR -1-endo/ 10 µM (FUJIFILM Wako Pure Chemical Corporation) | Miricitrin/20 µM (Sigma-Aldrich Co. LLC) |
| Comparative Example 5-4 | IWR -1-endo/ 10 µM (FUJIFILM Wako Pure Chemical Corporation) | K-252a/1nM (FUJIFILM Wako Pure Chemical Corporation) |

### <Example 3: Addition of TNKS Inhibitor and PKCβ Inhibitor to Suspension Culture of Human iPS Cell 201B7 Strain>

To check whether differentiation-suppressing effect is exerted when a PKCβ inhibitor other than Go6983, which was used in Example 1, is used, suspension culture was carried out in the following manner.

Suspension culture was carried out with the same procedure as in Comparative Example 4, except that a combination of a TNKS inhibitor and a PKCβ inhibitor shown in Table 4 was added to medium for seeding and medium for medium exchange in suspension culture.

**[Table 4]**

| | TNKS inhibitor | PKCβ inhibitor |
|---|---|---|
| | Name of reagent/final concentration | Name of reagent/final concentration |
| Example 3-1 | IWR -1-endo/ 10 µM (FUJIFILM Wako Pure Chemical Corporation) | Go6983/3 µM (FUJIFILM Wako Pure Chemical Corporation) |
| Example 3-2 | IWR -1-endo/ 10 µM (FUJIFILM Wako Pure Chemical Corporation) | GF109203X/1 µM (Tocris Bioscience) |
| Example 3-3 | IWR -1-endo/ 10 µM (FUJIFILM Wako Pure Chemical Corporation) | LY-333531/1 µM (Sigma-Aldrich Co. LLC) |

### <Example 4: Quantitative Real-Time PCR Analysis>

Quantitative real-time PCR analysis was performed with a procedure shown in the following. The cells on day 4 of culture in Reference Example 2, and the cells on day 8 of culture in Comparative Examples 4 and 5 and Example 3 were dissolved by using TRIzol (TM) Reagent (Thermo Fisher Scientific). From each solution resulting from dissolution with TRIzol (TM) Reagent, total RNA was isolated and purified by using a PureLink (R)RNA Mini kit (Thermo Fisher Scientific). The RNA purified was subjected to concentration measurement using a BioSpec-nano (Shimadzu Corporation), and RNA in an amount of 500 ng was separated. To the RNA separated, 2 µL of ReverTra Ace(R) qPCR RT Master mix (TOYOBO CO., LTD.) and Rnase Free dH₂O were added to prepare 10 µL of solution, and cDNA synthesis was performed by using a SimpliAmp Thermal Cycler (Thermo Fisher Scientific). The reaction conditions for the cDNA synthesis were such that reaction at 37°C for 15 minutes was followed by consecutive reactions of reaction at 50°C for 5 minutes and reaction at 98°C for 5 minutes, followed by cooling to 4°C. The synthesized cDNA solution was 100-fold diluted with 10 mM Tris-HCl pH 8.0 (NACALAI TESQUE, INC.), and added to a 384-well PCR plate (Thermo Fisher Scientific) at 5 µL/well. KOD SYBR (R) qPCR Mix (TOYOBO CO., LTD.), Forward primer prepared as 50 µM solution, Reverse primer prepared as 50 µM solution, and DEPC-treated water (NACALAI TESQUE, INC.) were mixed together at a ratio of 100:1:1:48, and this mixed solution was added to the 384-well PCR plate at 15 µL/well and mixed together. The primers used were those for GAPDH, OCT4, T, SOX17, and PAX6. The 384-well PCR plate was centrifuged to remove bubbles in the wells, and quantitative real-time PCR analysis was carried out by using a QuantStudio 7 Flex Real-Time PCR System (Thermo Fisher Scientific). Table 5 shows the reaction conditions.

**[Table 5]**

| Step | Temperature | Time | Number of cycles |
|---|---|---|---|
| 1 Initial modification | 98°C | 1 min | - |
| 2 Modification | 98°C | 15 sec | 5 cycle |
| 3 Annealing, elongation | 68°C | 30 sec | |
| 4 Modification | 98°C | 15 sec | 40 cycle |
| 5 Annealing | 60°C | 10 sec | |
| 6 Elongation | 68°C | 30 sec | |
| 7 Melting curve | 95°C | 15 sec | - |
| | 60°C | 1 min | |
| | 98°C | 15 sec | |

The nucleotide sequences of the primers used for the quantitative real-time PCR analysis are shown in the following.
ACTB (Forward): 5'-CCTCATGAAGATCCTCACCGA-3' (SEQ. ID. No.1)
ACTB (Reverse): 5'-TTGCCAATGGTGATGACCTGG-3' (SEQ. ID. No.2)
OCT4 (Forward): 5'-AGTGGGTGGAGGAAGCTGACAAC-3' (SEQ. ID. No.3)
OCT4 (Reverse): 5'-TCGTTGTGCATAGTCGCTGCTTGA-3' (SEQ. ID. No.4)
SOX2 (Forward): 5'-CACCAATCCCATCCACACTCAC-3' (SEQ. ID. No.5)
SOX2 (Reverse): 5'-GCAAAGCTCCTACCGTACCAC-3' (SEQ. ID. No.6)
NANOG (Forward): 5'-AGCCTCCAGCAGATGCAAGAACTC-3' (SEQ. ID. No.7)
NANOG (Reverse): 5'-TTGCTCCACATTGGAAGGTTCCCA-3' (SEQ. ID. No.8)
T (Forward): 5'-TCACAAAGAGATGATGGAGGAAC-3' (SEQ. ID. No.9)
T (Reverse): 5'-ACATGCAGGTGAGTTGTCAG-3' (SEQ. ID. No.10)
SOX17 (Forward): 5'-ATCTGCACTTCGTGTGCAAG-3' (SEQ. ID. No. 1 1)
SOX17 (Reverse): 5'-GAGTCTGAGGATTTCCTTAGCTC-3' (SEQ. ID. No.12)
PAX6 (Forward): 5'-AGGAATGGACTTGAAACAAGG-3' (SEQ. ID. No. 13)
PAX6 (Reverse): 5'-GCAAAGCTTGTTGATCATGG-3' (SEQ. ID. No.14)

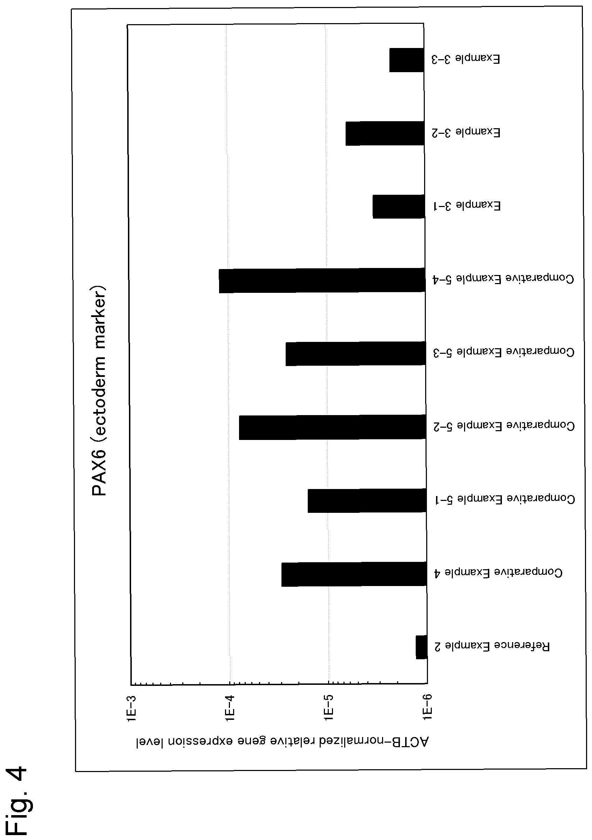

Table 6 and Figure 4 show the results of measurement of gene expression.

**[Table 6]**

| | ACTB-normalized relative gene expression level of PAX6(2^{-ΔCt}) |
|---|---|
| Reference Example 2 | 1.29 × 10⁻⁶ |
| Comparative Example 4 | 2.92 × 10⁻⁵ |
| Comparative Example 5-1 | 1.57 × 10⁻⁵ |
| Comparative Example 5-2 | 7.69 × 10⁻⁵ |
| Comparative Example 5-3 | 2.59 × 10⁻⁵ |
| Comparative Example 5-4 | 1.21 × 10⁻⁴ |
| Example 3-1 | 3.33 × 10⁻⁶ |
| Example 3-2 | 6.16 × 10⁻⁶ |
| Example 3-3 | 2.20 × 10⁻⁶ |

As shown in Table 6 and Figure 4, the expression level of PAX6 for Comparative Example 4, in which suspension culture was performed, was higher than that in the adherent culture (Reference Example 2), indicating the occurrence of spontaneous differentiation into ectoderm. For Comparative Examples 5-1 to 5-4, in which a TNKS inhibitor and a PKC inhibitor (that inhibits the activity of a PKC isozyme other than PKCβ) were added, the expression levels of PAX6 were comparable to that for Comparative Example 4. For Examples 3-1 to 3-3, in which a TNKS inhibitor and a PKCβ inhibitor were added, the expression levels of PAX6 were lower than that for Comparative Example 4, and comparable to that for Reference Example 2.

All the four PKC inhibitors added in Comparative Examples 5-1 and 5-4 lack inhibitory effect on PKCβ. On the other hand, LY-333531, which was added in Example 3, is a selective inhibitor for PKCβ, and Go6983 and GF109203X are broad-spectrum inhibitors that inhibit the activities of a plurality of PKC isozymes including PKCβ. From those results, it was revealed that inhibition of PKCβ is effective for suppression of spontaneous differentiation into ectoderm, and PKCβ inhibitors are useful as differentiation inhibitors for the differentiation of pluripotent stem cells into ectoderm.

### <Reference Example 3: Preparation of Cells for Flow Cytometry Analysis>

For flow cytometry analysis of the undifferentiation of an adherent-cultured cell population, culture was performed with the same procedure as in Reference Example 2 for 4 days to prepare cells.

### <Comparative Example 6: Preparation of Cells for Flow Cytometry Analysis>

For flow cytometry analysis of the undifferentiation of a suspension-cultured cell population, culture was performed with the same procedure as in Comparative Example 4 for 8 days to prepare cells.

### <Comparative Example 7: Preparation of Cells for Flow Cytometry Analysis>

For flow cytometry analysis of the undifferentiation of a cell population suspension-cultured with addition of a TNKS inhibitor and a PKC inhibitor (that inhibits the activity of a PKC isozyme other than PKCβ), culture was performed with the same procedure as in Comparative Example 5 for 8 days to prepare cells. Table 7 shows combinations of a TNKS inhibitor and a PKC inhibitor (that inhibits the activity of a PKC isozyme other than PKCβ) used.

**[Table 7]**

| | TNKS inhibitor | PKC inhibitor |
|---|---|---|
| | Name of reagent/final concentration | Name of reagent/final concentration |
| Comparative Example 7-1 | IWR-1-endo/10 µM | Staurosporine/1nM |
| Comparative Example 7-2 | IWR-1-endo/10 µM | ZIP/1 µM |
| Comparative Example 7-3 | IWR-1-endo/10 µM | Miricitrin/20 µM |
| Comparative Example 7-4 | IWR-1-endo/10 µM | K-252a/1nM |

### <Example 5: Preparation of Cells for Flow Cytometry Analysis>

For flow cytometry analysis of the undifferentiation of a cell population suspension-cultured with addition of a TNKS inhibitor and a PKCβ inhibitor, culture was performed with the same procedure as in Example 3-2 or 3-3 for 8 days to prepare cells. Table 8 shows combinations of a TNKS inhibitor and a PKCβ inhibitor used.

**[Table 8]**

| | TNKS inhibitor | PKCβ inhibitor |
|---|---|---|
| | Name of reagent/final concentration | Name of reagent/final concentration |
| Example 5-1 | IWR-1-endo/10 µM | GF109203X/1 µM |
| Example 5-2 | IWR-1-endo/10 µM | LY-333531/1 µM |

### <Example 6: Flow Cytometry Analysis>

Flow cytometry analysis was performed with a procedure shown in the following. The cells obtained in Reference Example 3, Comparative Example 6, Comparative Example 7, and Example 5 were treated with Accutase, and dispersed into single cells by pipetting. The cells were washed with PBS (phosphate-buffered saline). Thereafter, the cells were fixed with 4% PFA (paraformaldehyde) at room temperature for 20 minutes, washed three times with PBS, and subjected to permeabilization with cold methanol at -20°C overnight. After washing three times with PBS, the cells were blocked with 3% FBS (fetal bovine serum)/PBS at room temperature for 1 hour. Thereafter, each cell sample was divided into two, which were each resuspended in 50 µL. Fluorescence-labeled anti-OCT4, anti-SOX2, and anti-NANOG antibodies were added to one suspension and mixed together, and fluorescence-labeled isotype control antibodies were added to the other suspension and mixed together, and staining was performed at 4°C under shading for 1 hour. Table 9 shows the antibodies used and the loadings thereof.

**[Table 9]**

| | Manufacturer, model number | Loading |
|---|---|---|
| Fluorescence-labeled anti-OCT4 antibody | BioLegend, 653704 | 5 µL |
| Fluorescence-labeled anti-SOX2 antibody | BioLegend, 656110 | 5 µL |
| Fluorescence-labeled anti-NANOG antibody | BioLegend, 674010 | 5 µL |
| Fluorescence-labeled OCT4 isotype control antibody | BioLegend, 400314 | 0.625 µL |
| Fluorescence-labeled SOX2 isotype control antibody | BioLegend, 400129 | 0.625 µL |
| Fluorescence-labeled Nanog isotype control antibody | BioLegend, 400130 | 0.625 µL |

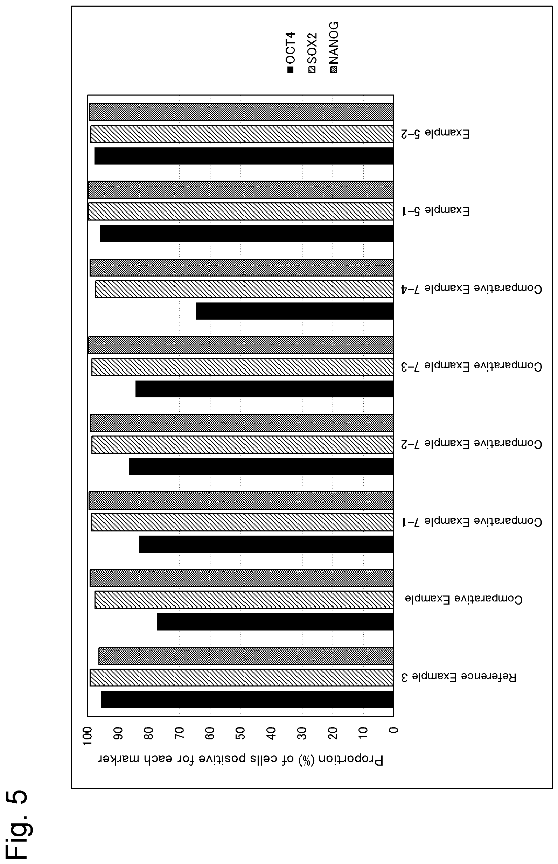

After washing once with 3% FBS (fetal bovine serum)/PBS, the cells passed through a cell strainer were analyzed with a Guava easyCyte 8HT (Luminex Corporation). For each sample treated with the isotype control antibodies, all regions where the proportion of cell populations with higher fluorescence intensity among cell populations extracted from the FSC/SSC dot plot was 1.0% or lower were selected. For the sample treated with the anti-OCT4, anti-SOX2, and anti-NANOG antibodies, the percentages of cells included in the regions in the cell populations extracted from the FSC/SSC dot plot were calculated, and the resultants were used as the proportions of cells positive for OCT4, SOX2, and NANOG. Table 10 and Figure 5 shows the results.

**[Table 10]**

| | Proportion (%) of cells positive for OCT4 | Proportion (%) of cells positive for SOX2 | Proportion (%) of cells positive for NANOG |
|---|---|---|---|
| Reference Example 3 | 95.5 | 99.1 | 96.2 |
| Comparative Example 6 | 77.1 | 97.5 | 99.1 |
| Comparative Example 7-1 | 83.1 | 98.8 | 99.4 |
| Comparative Example 7-2 | 86.4 | 98.5 | 99.0 |
| Comparative Example 7-3 | 84.2 | 98.5 | 99.6 |
| Comparative Example 7-4 | 64.4 | 97.3 | 99.1 |
| Example 5-1 | 95.9 | 99.5 | 99.5 |
| Example 5-2 | 97.6 | 98.9 | 99.3 |

As shown in Table 10 and Figure 5, the proportions of cells positive for the undifferentiation markers OCT4, SOX2, and NANOG were 90% or higher for Reference Example 3, in which adherent culture was performed; thus, pluripotent stem cell populations with the undifferentiated state maintained were obtained. For Comparative Example 6, in which suspension culture was performed, and Comparative Example 7, in which suspension culture was performed with addition of a TNKS inhibitor and a PKC inhibitor, on the other hand, the proportions of cells positive for OCT4 were lower, indicating the appearance of spontaneously differentiated cell populations and thus failure in maintaining the undifferentiated state of pluripotent stem cells. For Example 5, in which suspension culture was performed with addition of a TNKS inhibitor and a PKCβ inhibitor, the proportions of cells positive for OCT4, SOX2, and NANOG were 90% or higher, suggesting that cell populations with the undifferentiated state maintained by suppressing the spontaneous differentiation were obtained. Also from these results, it was revealed that suspension culture using a TNKS inhibitor and a PKCβ inhibitor can provide a pluripotent stem cell population with the undifferentiated state maintained by suppressing the spontaneous differentiation.

### <Reference Example 4: Adherent Culture of Human iPS Cell 201B7 Strain>

Adherent culture was carried out with the same procedure as in Reference Example 2.

### <Comparative Example 8: Suspension Culture of Human iPS Cell 201B7 Strain>

Human iPS cell 201B7 strain subjected to adherent culture with the same procedure as in Reference Example 2 was treated with Accutase for 5 minutes to detach from the culture surface, and dispersed into single cells by pipetting. The cells were suspended in StemFit (R) AK02N medium containing Y-27632 with a final concentration of 10 µM, and some of the cells were stained with trypan blue and the number of viable cells was counted. StemFit (R) AK02N medium containing Y-27632 with a final concentration of 10 µM was applied to the cell suspension to prepare a cell suspension containing 2 × 10⁵ cells per 1 mL. Into a 12-well plate for suspension culture (Sumitomo Bakelite Co., Ltd.), 1 mL of the cell suspension was seeded per well. On a rotary shaker, the plate into which the cells had been seeded was rotated along a circle having a rotation width (diameter) of 25 mm in the horizontal plane at a speed of 98 rpm, and suspension culture was performed under an environment at 37°C and 5% CO₂. Subculture was performed on day 5 of culture as the day on which the cells were seeded was defined as day 0 of culture, and suspension culture was performed until day 10 of culture. Medium exchange was performed every day. Each medium exchange was performed in such a manner that the whole volume of medium containing a cell aggregate was collected into a centrifuge tube, left to stand for about 5 minutes to precipitate the cell aggregate, the culture supernatant was then removed, and the resultant was gently resuspended in StemFit (R) AK02N medium, which was returned into the original well. In medium exchange, Y-27632 was added to the medium to give a final concentration of 5 µM on day 1 and 6 of culture, and Y-27632 was added to the medium to give a final concentration of 2 µM on day 2 and 7 of culture. On day 5 of culture, each cell aggregate and culture supernatant were collected into a centrifuge tube, left to stand for about 5 minutes to precipitate the cell aggregate, and the culture supernatant was removed. After 1 mL of Accutase was added to each cell aggregate for treatment at 37°C for 10 minutes, the cell aggregate was dispersed into single cells by pipetting. The cells were suspended in StemFit (R) AK02N medium containing Y-27632 with a final concentration of 10 µM, and some of the cells were stained with trypan blue and the number of viable cells was counted. StemFit (R) AK02N medium containing Y-27632 with a final concentration of 10 µM was applied to the cell suspension to prepare a cell suspension containing 2 × 10⁵ cells per 1 mL, and the cells were seeded in the same manner as on day 0 of culture to continue suspension culture.

### <Comparative Example 9: Addition of PORCN Inhibitor and PKCβ Inhibitor to Suspension Culture of Human iPS Cell 201B7 Strain>

The TNKS inhibitor used in Example 1 is known to have inhibitor effect on WNT signaling. In view of this, to check whether a Porcupine (PORCN) inhibitor, which has inhibitory effect on WNT signaling like the TNKS inhibitor, similarly exhibits differentiation-suppressing effect when being used, suspension culture was carried out in the following manner.

Suspension culture was carried out with the same procedure as in Comparative Example 8, except that, to medium for seeding and medium for medium exchange in suspension culture, IWP-2 (FUJIFILM Wako Pure Chemical Corporation) or WNT-C59 (Cayman Chemical Company) was added as a PORCN inhibitor and GF109203X or LY-333531 was added as a PKCβ inhibitor.

### <Example 7: Addition of TNKS Inhibitor and PKCβ Inhibitor to Suspension Culture of Human iPS Cell 201B7 Strain>

To check whether a TNKS inhibitor other than IWR-1-endo, which was used in Example 1, similarly exhibits differentiation-suppressing effect when being used, suspension culture was carried out in the following manner.

Suspension culture was carried out with the same procedure as in Comparative Example 8, except that, to medium for seeding and medium for medium exchange in suspension culture, IWR-1-endo or XAV939 (FUJIFILM Wako Pure Chemical Corporation) was added as a TNKS inhibitor and GF109203X or LY-333531 was added as a PKCβ inhibitor.

### <Example 8: Quantitative Real-Time PCR Analysis>

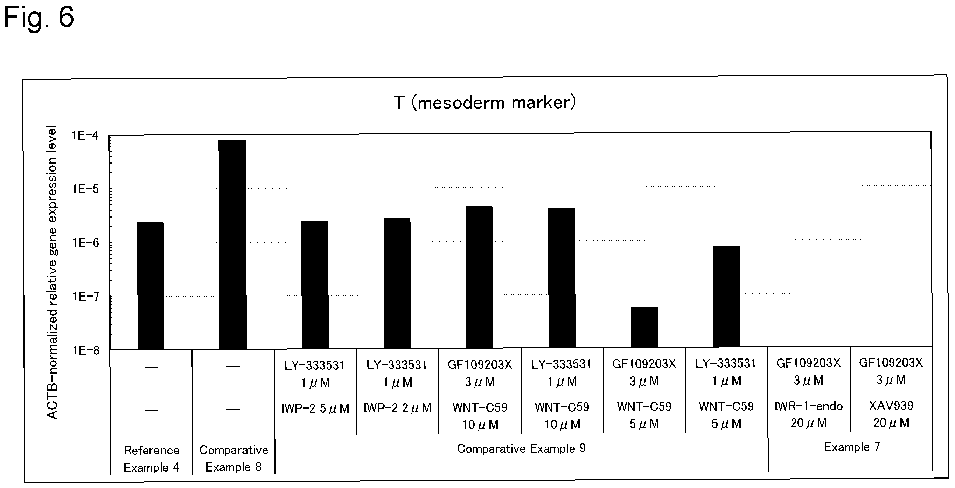

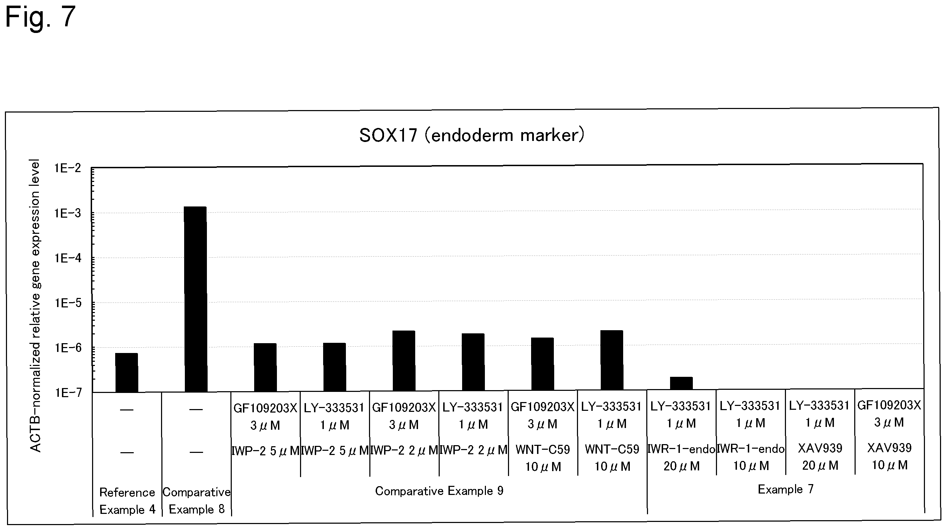

Quantitative real-time PCR analysis was carried out with the same procedure as in Example 4 for the cells on day 4 of culture in Reference Example 4, and the cells on day 10 of culture in Comparative Example 8, Comparative Example 9, and Example 7. Tables 11 and 12 and Figures 6 and 7 show the results of measurement of gene expression.

**[Table 11]**

| | PORCN inhibitor or TNKS inhibitor | PKCβ inhibitor | ACTB-normalized relative gene expression level of T(2^{- ΔCt}) |
|---|---|---|---|
| Reference Example 4 | - | - | 2.38 × 10⁻⁶ |
| Comparative Example 8 | - | - | 7.78 × 10⁻⁵ |
| Comparative Example 9 | IWP-2 5 µM | LY-333531 1 µM | 2.41 × 10⁻⁶ |
| | IWP-2 2 µM | LY-333531 1 µM | 2.66 × 10⁻⁶ |
| | WNT-C59 10 µM | GF109203X 3 µM | 4.31 × 10⁻⁶ |
| | WNT-C59 10 µM | LY-333531 1 µM | 3.91 × 10⁻⁶ |
| | WNT-C59 5 µM | GF 109203X 3 µM | 5.46 × 10⁻⁸ |
| | WNT-C59 5 µM | LY-333531 1 µM | 7.51 × 10⁻⁷ |
| Example 7 | IWR-1-endo 20 µM | GF109203X 3 µM | N.D.(Not detected) |
| | XAV939 20 µM | GF 109203X 3 µM | N.D. |

**[Table 12]**

| | PORCN inhibitor or TNKS inhibitor | PKCβ inhibitor | ACTB-normalized relative gene expression level of SOX17(2^{-Δct}) |
|---|---|---|---|
| Reference Example 4 | - | - | 7.25 × 10⁻⁷ |
| Comparative Example 8 | - | - | 1.33 × 10⁻³ |
| Comparative Example 9 | IWP-2 5 µM | GF 109203X 3 µM | 1.16 × 10⁻⁶ |
| | IWP-2 5 µM | LY-333531 1 µM | 1.17 × 10⁻⁶ |
| | IWP-2 2 µM | GF 109203X 3 µM | 2.15 × 10⁻⁶ |
| | IWP-2 2 µM | LY-333531 1 µM | 1.82 × 10⁻⁶ |
| | WNT-C59 10 µM | GF 109203X 3 µM | 1.45 × 10⁻⁶ |
| | WNT-C59 10 µM | LY-333531 1 µM | 2.08 × 10⁻⁶ |
| Example 7 | IWR-1-endo 20 µM | LY-333531 1 µM | 1.89 × 10⁻⁷ |
| | IWR-1-endo 10 µM | LY-333531 1 µM | N.D.(Not detected) |
| | XAV939 20 µM | LY-333531 1 µM | N.D. |
| | XAV939 10 µM | GF 109203X 3 µM | N.D. |

As shown in Tables 11 and 12 and Figures 6 and 7, the expression levels of T, SOX17, and PAX6 were low and the undifferentiated state of pluripotent stem cells was maintained for Reference Example 4, in which adherent culture was performed. For Comparative Example 8, in which suspension culture was performed, on the other hand, increased expression levels were achieved for T and SOX17, indicating the occurrence of spontaneous differentiation into mesoderm and endoderm and failure in maintaining the undifferentiated state of pluripotent stem cells. For Comparative Example 9, in which suspension culture was performed with addition of a PORCN inhibitor and a PKCβ inhibitor, the expression levels of T and SOX17 were lower than those for Comparative Example 8, indicating that the PORCN inhibitors suppress spontaneous differentiation into mesoderm and endoderm. For Example 7, in which suspension culture was performed with addition of a TNKS inhibitor and a PKCβ inhibitor, the expression levels of T and SOX17 were much lower than those for Comparative Example 9, indicating that the TNKS inhibitors are capable of suppressing spontaneous differentiation into mesoderm and endoderm more strongly than the PORCN inhibitors. While the PORCN inhibitors and the TNKS inhibitors each have inhibitory effect on WNT signaling, it was revealed that the TNKS inhibitors are more effective when being combined with a PKCβ inhibitor for suppression of spontaneous differentiation into mesoderm and endoderm.

### <Reference Example 5: Adherent Culture of Human iPS Cell 201B7 Strain>

Adherent culture was carried out with the same procedure as in Reference Example 2.

### <Comparative Example 10: Suspension Culture of Human iPS Cell 201B7 Strain>

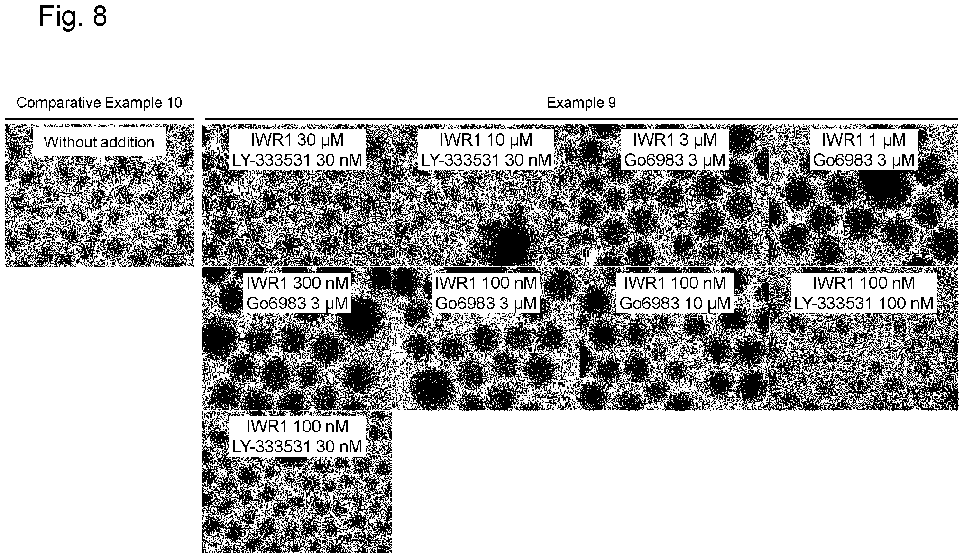

Suspension culture was carried out with the same procedure as in Comparative Example 8. On day 8 of culture, phase-contrast images of cell aggregates were acquired by using a phase-contrast microscope. Figure 8 shows phase-contrast images of cell aggregates. The formation of cell aggregates was confirmed from Figure 8.

### <Example 9: Examination of Concentrations of TNKS Inhibitor and PKCβ Inhibitor in Suspension Culture of Human iPS Cell 201B7 Strain>

Suspension culture was carried out with the same procedure as in Comparative Example 8, except that, to medium for seeding and medium for medium exchange in suspension culture, IWR-1-endo was added as a TNKS inhibitor to give a final concentration of 30 µM, 10 µM, 3 µM, 1 µM, 300 nM, or 100 nM, and Go6983 or LY-333531 was added as a PKCβ inhibitor to give a final concentration of 10 µM, 100 nM, or 30 nM.

### <Example 10: Quantitative Real-Time PCR Analysis>

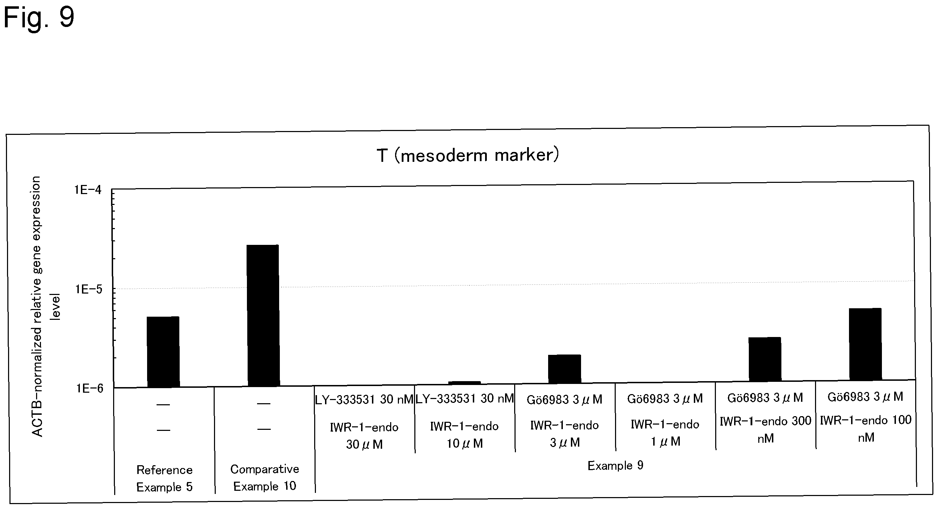

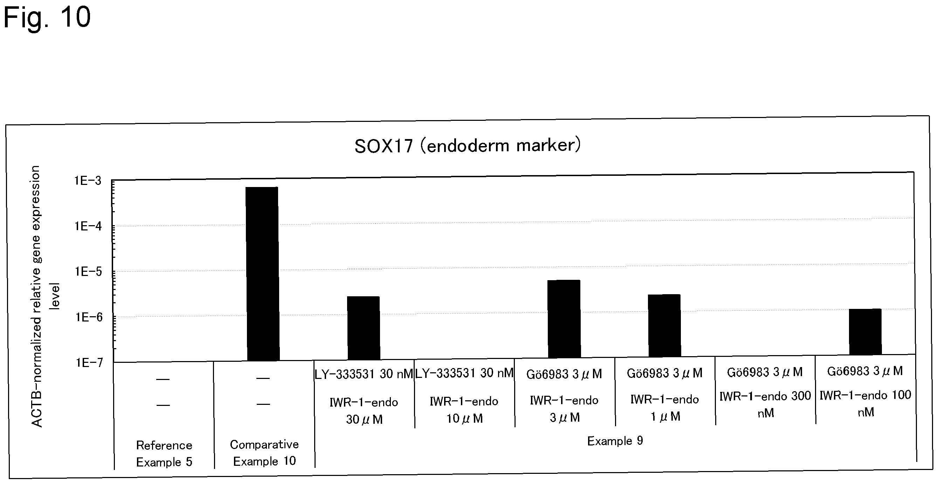

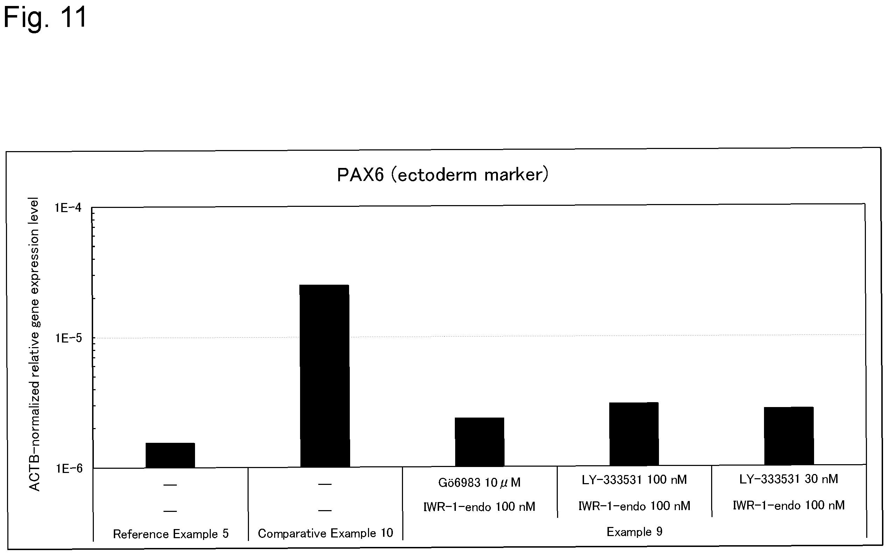

Quantitative real-time PCR analysis was carried out with the same procedure as in Example 4 for the cells on day 4 of culture in Reference Example 5, and the cells on day 10 of culture in Comparative Example 10 and Example 9. Tables 13 and 14 and Figures 9 to 11 show the results of measurement of gene expression.

**[Table 13]**

| | TNKS inhibitor | PKCβ inhibitor | ACTB-normalized relative gene expression level (2^{-ΔCt}) | |
|---|---|---|---|---|
| | | | T | SOX17 |
| Reference Example 5 | - | - | 5.10 × 10⁻⁶ | N.D. |
| Comparative Example 10 | - | - | 2.61 × 10⁻⁵ | 6.36 × 10⁻⁴ |
| Example 9 | IWR-1-endo 30 µM | LY-333531 30 nM | N.D. | 2.42 × 10⁻⁶ |
| | IWR-1-endo 10 µM | LY-333531 30 nM | 1.06 × 10⁻⁶ | N.D. |
| | IWR-1-endo 3 µM | Go6983 3 µM | 1.93 × 10⁻⁶ | 4.99 × 10⁻⁶ |
| | IWR-1-endo 1 µM | Go6983 3 µM | N.D. | 2.27 × 10⁻⁶ |
| | IWR-1-endo 300 nM | Go6983 3 µM | 2.76 × 10⁻⁶ | N.D. |
| | IWR-1-endo 100 nM | Go6983 3 µM | 5.26 × 10⁻⁶ | 9.93 × 10⁻⁷ |

**[Table 14]**

| | TNKS inhibitor | PKCβ inhibitor | ACTB-normalized relative gene expression level (2^{-ΔCt}) |
|---|---|---|---|
| | | | PAX6 |
| Reference Example 5 | - | - | 1.55 × 10⁻⁶ |
| Comparative Example 10 | - | - | 2.47 × 10⁻⁵ |
| Example 9 | IWR-1-endo 100 nM | Go6983 10 µM | 2.34 × 10⁻⁶ |
| | IWR-1-endo 100 nM | LY-333531 100 nM | 3.02 × 10⁻⁶ |
| | IWR-1-endo 100 nM | LY-333531 30 nM | 2.76 × 10⁻⁶ |

As shown in Tables 13 and 14 and Figures 9 to 11, the expression levels of T, SOX17, and PAX6 were low and the undifferentiated state of pluripotent stem cells was maintained for Reference Example 5, in which adherent culture was performed. For Comparative Example 10, in which suspension culture was performed, on the other hand, increased expression levels were achieved for T, SOX17, and PAX6, indicating the occurrence of spontaneous differentiation into mesoderm, endoderm, and ectoderm and failure in maintaining the undifferentiated state of pluripotent stem cells. For Example 9, in which suspension culture was performed with addition of a TNKS inhibitor and a PKCβ inhibitor, the expression levels of T and SOX17 were lower than those for Comparative Example 10 and the spontaneous differentiation into mesoderm and endoderm was suppressed when the TNKS inhibitor within a concentration rage of 30 µM or less and 100 nM or more, as final concentration, was added. For Example 9, the expression level of PAX6 was lower than that for Comparative Example 10 and the spontaneous differentiation into ectoderm was suppressed when a PKCβ inhibitor within a concentration rage of 10 µM or less and 30 nM or more, as final concentration, was added.

### <Comparative Example 11: Addition of TNKS Inhibitor and PKCβ Inhibitor in Adherent Culture of Human iPS Cell 201B7 Strain>

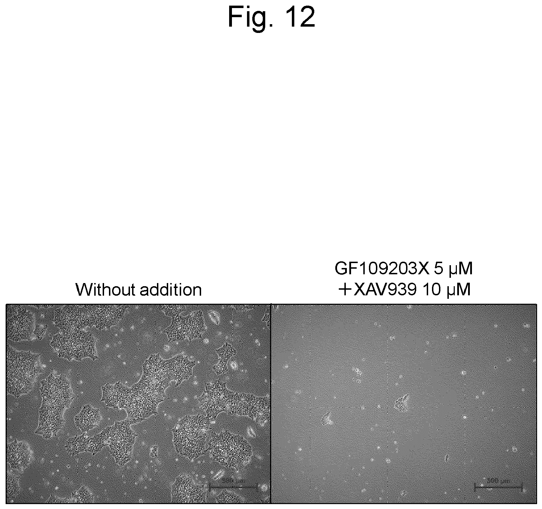

Adherent culture was carried out in two patterns: under conditions such that, in Reference Example 2, XAV939 and GF109203X were added to medium for seeding and medium for medium exchange in adherent culture to give final concentrations of 10 µM and 5 µM, respectively; and under conditions without involving the addition. Figure 12 shows phase-contrast images of cells on day 4 of culture. It was confirmed from Figure 12 that most cells were killed under the conditions involving addition of XAV939 and GF109203X. On day 4 of culture, the cells were treated with Accutase for 5 minutes to detach from the culture surface, and dispersed into single cells by pipetting. The cells were suspended in StemFit (R) AK02N medium containing Y-27632 with a final concentration of 10 µM, and some of the cells were stained with trypan blue and the number of viable cells was counted. As the number of viable cells on day 4 of culture over the number of seeded viable cells was defined as the amplification efficiency, the amplification efficiency under the conditions involving addition of XAV939 and GF109203X was 2.11 × 10⁻². This demonstrated that addition of a TNKS inhibitor and a PKCβ inhibitor adversely affects the survival and proliferation of pluripotent stem cells in adherent culture.

### <Example 11: Addition of TNKS Inhibitor and PKCβ Inhibitor in Suspension Culture of Human iPS Cell 201B7 Strain>

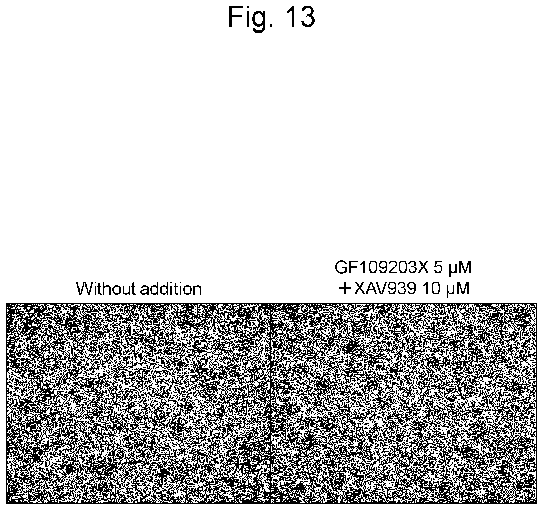

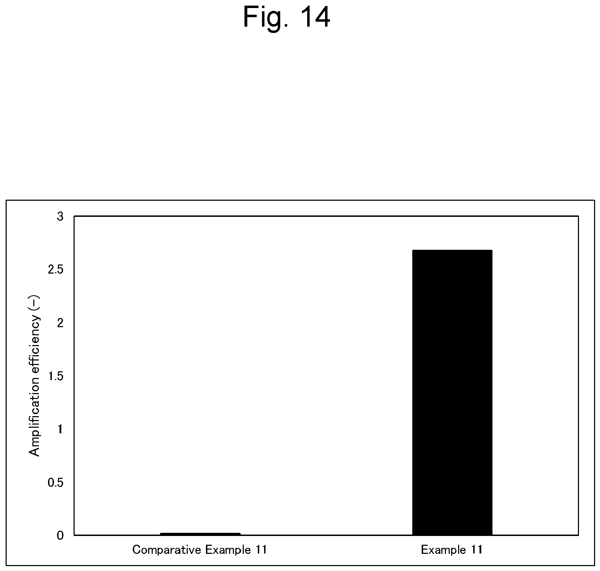

Suspension culture was carried out in two patterns: under conditions such that, in Comparative Example 4, XAV939 and GF109203X were added to medium for seeding and medium for medium exchange in adherent culture to give final concentrations of 10 µM and 5 µM, respectively; and under conditions without involving the addition. Figure 13 shows phase-contrast images of cells on day 4 of culture. It was confirmed from Figure 13 that, under the conditions involving addition of XAV939 and GF109203X, cell aggregates were formed as under the conditions without addition, and exhibited the same form. On day 4 of culture, the cells were treated with Accutase for 10 minutes for detachment, and dispersed into single cells by pipetting. The cells were suspended in StemFit (R) AK02N medium containing Y-27632 with a final concentration of 10 µM, and some of the cells were stained with trypan blue and the number of viable cells was counted. The amplification efficiency was calculated in the same manner as in Comparative Example 9, and the amplification efficiency under the conditions involving addition of XAV939 and GF109203X was found to be 2.68. Figure 14 shows comparison of the amplification efficiencies for Comparative Example 11 and Example 11. It was revealed from those results that significant improvement is provided in terms of adverse effect on survival and proliferation, as found for the adherent culture in Comparative Example 11, by addition of a TNKS inhibitor and a PKCβ inhibitor as in Example 11.

### <Comparative Example 12: Long-Term Suspension Culture of Human iPS Cell 1231A3 Strain>

Human iPS cell 1231A3 strain subjected to adherent culture with the same procedure as in Reference Example 2 was treated with Accutase (Innovative Cell Technologies, Inc.) for 5 minutes to detach from the culture surface, and dispersed into single cells by pipetting. The cells were suspended in StemFit (R) AK02N medium containing Y-27632 with a final concentration of 10 µM, and some of the cells were stained with trypan blue and the number of viable cells was counted. StemFit (R) AK02N medium containing Y-27632 with a final concentration of 10 µM was applied to the cell suspension to prepare a cell suspension containing 2 × 10⁵ cells per 1 mL. Into a 6-well plate for suspension culture, 4 mL of the cell suspension was seeded per well. On a rotary shaker, the plate into which the cells had been seeded was rotated along a circle having a rotation width (diameter) of 25 mm in the horizontal plane at a speed of 90 rpm, and suspension culture was performed under an environment at 37°C and 5% CO₂.

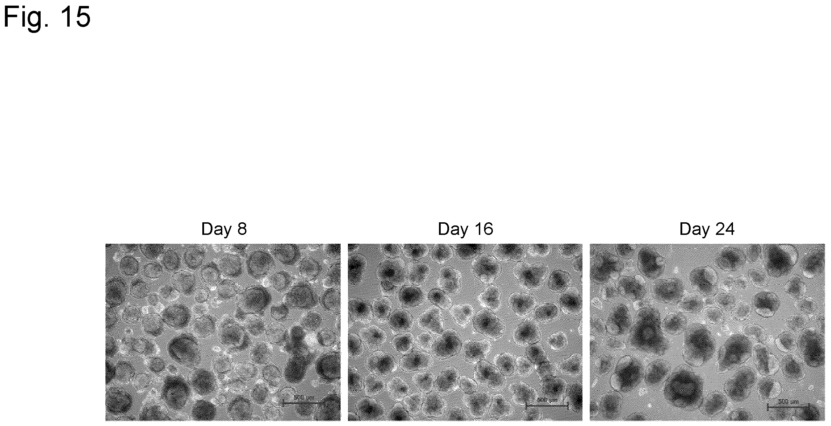

Subculture was performed on day 4, 8, 16, and 20 of culture as the day on which the cells were seeded was defined as day 0 of culture, and suspension culture was performed until day 24 of culture. Medium exchange was performed every day. Each medium exchange was performed in such a manner that the whole volume of medium containing a cell aggregate was first collected into a centrifuge tube, left to stand for about 5 minutes to precipitate the cell aggregate, the culture supernatant was then removed, and the resultant was gently resuspended in StemFit (R) AK02N medium, which was returned into the original well. In medium exchange, Y-27632 was added to the medium to give a final concentration of 5 µM on day 1, 5, 9, 13, 17, and 21 of culture, and Y-27632 was added to the medium to give a final concentration of 2 µM on day 2, 6, 10, 14, 18, and 22 of culture. On day 4, 8, 12, 16, and 20 of culture, each cell aggregate and culture supernatant were collected into a centrifuge tube, left to stand for about 5 minutes to precipitate the cell aggregate, and the culture supernatant was removed. After 1 mL of Accutase was added to each cell aggregate for treatment at 37°C for 10 minutes, the cell aggregate was dispersed into single cells by pipetting. The cells were suspended in StemFit (R) AK02N medium containing Y-27632 with a final concentration of 10 µM, and some of the cells were stained with trypan blue and the number of viable cells was counted. StemFit (R) AK02N medium containing Y-27632 with a final concentration of 10 µM was applied to the cell suspension to prepare a cell suspension containing 2 × 10⁵ cells per 1 mL, and the cells were seeded in the same manner as on day 0 of culture to continue suspension culture. On day 8, 16, and 24 of culture, phase-contrast images of cell aggregates were acquired by using a phase-contrast microscope. Figure 15 shows phase-contrast images of cell aggregates. The formation of cell aggregates was confirmed from Figure 15, but the shapes were irregular.

### <Example 12: Addition of TNKS Inhibitor and PKCβ Inhibitor in Long-Term Suspension Culture of Human iPS Cell 1231A3 Strain>

Culture was performed in the same manner as in Comparative Example 12, except that 20 µM IWR-1-endo as a TNKS inhibitor and 1 µM LY-333531 as a PKCβ inhibitor were added to medium in suspension culture, and that, immediately before medium exchange and subculture, sampling of culture supernatant was performed and measurement of lactic acid concentration was performed by using the multifunctional biosensor BF-7D manufactured by Oji Scientific Instruments.

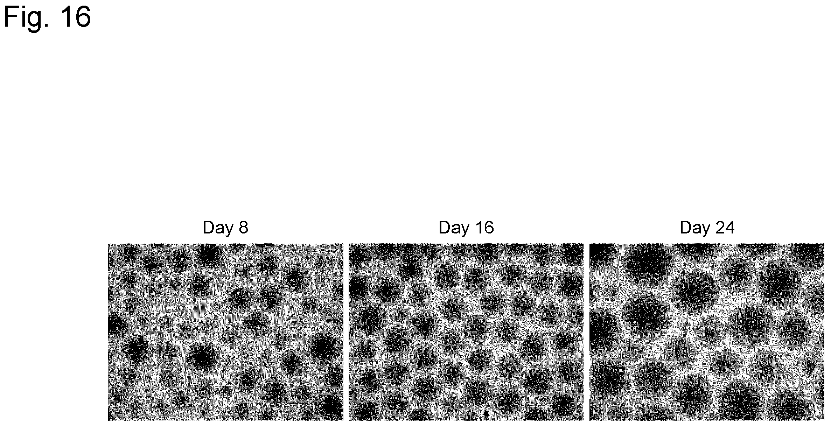

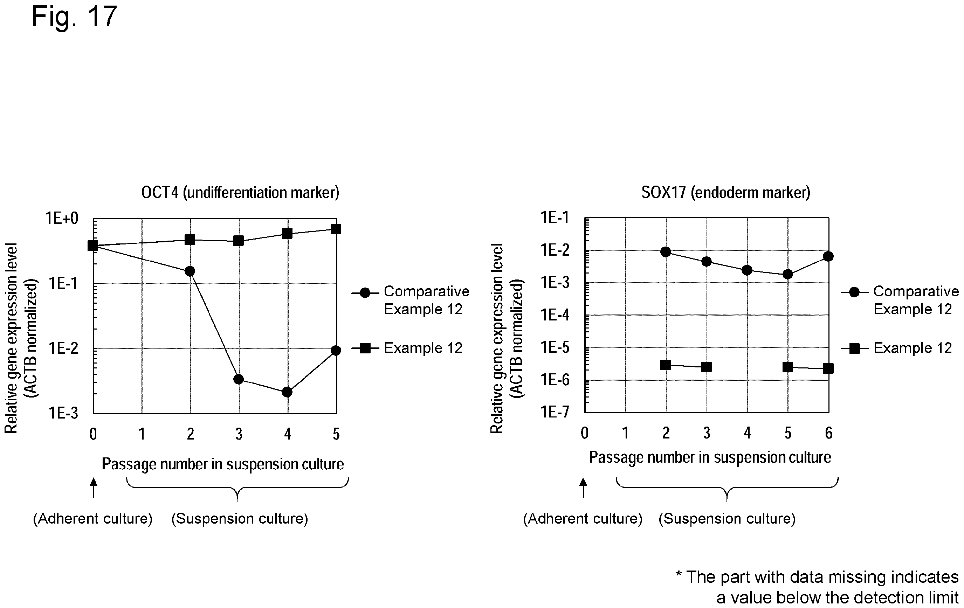

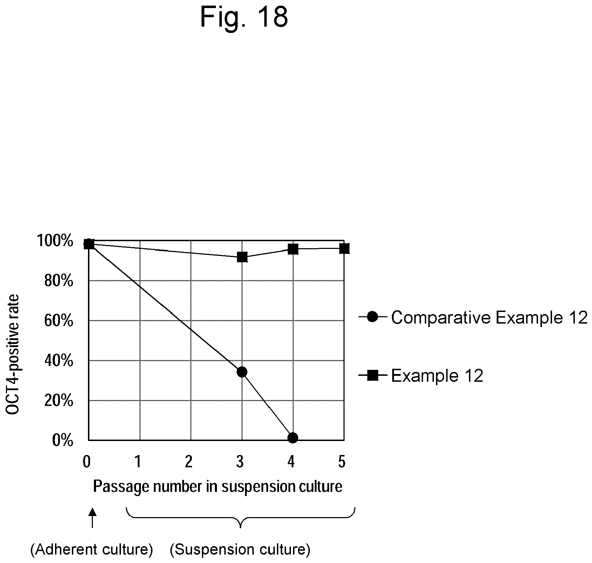

Figure 16 shows phase-contrast images of cell aggregates. The formation of spherical, well-shaped cell aggregates was confirmed from Figure 16. Figure 17 shows transition of results of quantitative real-time PCR analysis (see Example 4 for primers and analysis conditions used) for Comparative Example 12 and Example 12. As shown in Figure 17, it was found that the relative gene expression level of the undifferentiation marker OCT4 was kept high even in long-term culture for Example 12. It is understood that the relative gene expression level of the endoderm marker SOX17 was kept low even in long-term culture for Example 12. Figure 18 shows transition of the positive rates for the undifferentiation marker OCT4 for Comparative Example 12 and Example 12. It is understood from Figure 18 that the positive rate for the undifferentiation marker OCT4 was kept high even in long-term culture for Example 12.

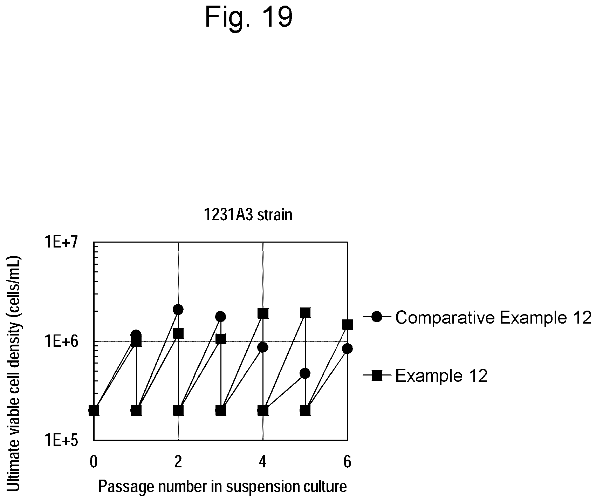

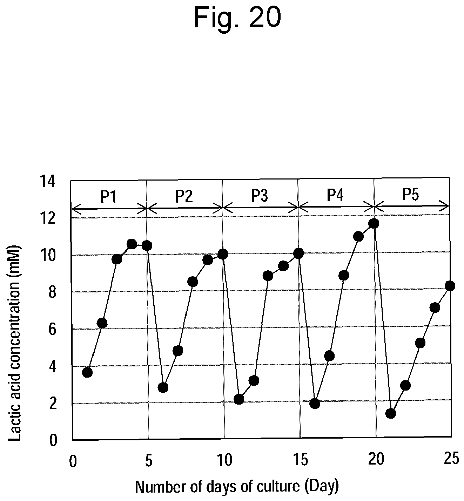

The results shown in Figures 15 to 18 for Comparative Example 12 and Example 12 revealed that use of medium containing a TNKS inhibitor and a PKCβ inhibitor allows pluripotent stem cells to maintain the undifferentiation even when the pluripotent stem cells are subjected to suspension culture for a long period of time. Figure 19 shows transition of ultimate viable cell density for Comparative Example 12 and Example 12. It was found from Figure 19 that the proliferative ability was successfully maintained for Example 12 even after pluripotent stem cells were subjected to suspension culture for a long period of time. Figure 20 shows the results of measurement of lactic acid concentration in medium immediately before medium exchange and subculture for Example 12. It was found from Figure 20 that the lactic acid concentration in medium immediately before subculture was over 10 mM, being as high as about 12 mM It was found from the above results shown in Figures 17 to 20 that use of medium containing a TNKS inhibitor and a PKCβ inhibitor allows pluripotent stem cells to maintain the undifferentiation and proliferative capacity even when the lactic acid concentration in medium is relatively high.

### <Example 13: Long-Term Suspension Culture Using Stirring Reactor>

Into a culture vessel for suspension culture, 30 mL of cell suspension was seeded by using a single-use bioreactor (30 mL) for iPS cell culture, manufactured by ABLE Corporation, and subjected to suspension culture until day 15 of culture with stirring at a blade tip speed of 135 rpm, that is, 0.23 m/s, and subculture on day 3, 6, 9, and 12 of culture. Culture was performed in the same manner as in Example 12, except that, in medium exchange in the present example, Y-27632 was added to the medium to give a final concentration of 6.9 µM on day 1, 4, 7, 10, and 13 of culture, and Y-27632 was added to the medium to give a final concentration of 3.71 µM on day 2, 5, 8, 11, and 14 of culture.

### <Reference Example 6: Long-Term Adherent Culture of Human iPS Cell 1231A3 Strain>

Human iPS cell 1231A3 strain subjected to adherent culture with the same procedure as in Reference Example 2 was treated with Accutase (Innovative Cell Technologies, Inc.) for 5 minutes to detach from the culture surface, and dispersed into single cells by pipetting. Thereafter, culture and subculture were repeatedly performed with the same procedure as in Reference Example 2, except that the number of days of culture and passage number were the same as those in Example 13.

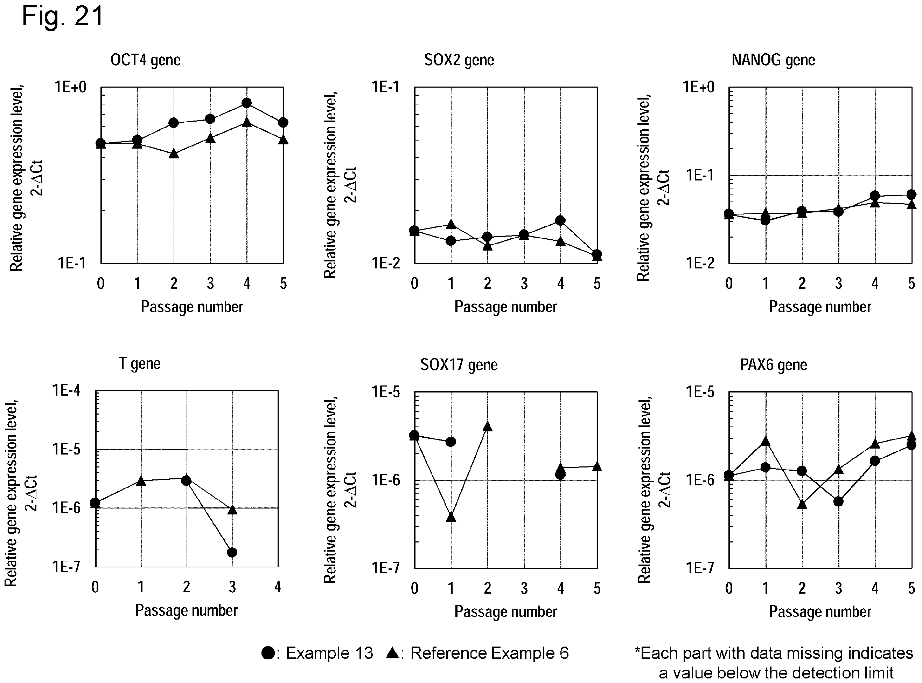

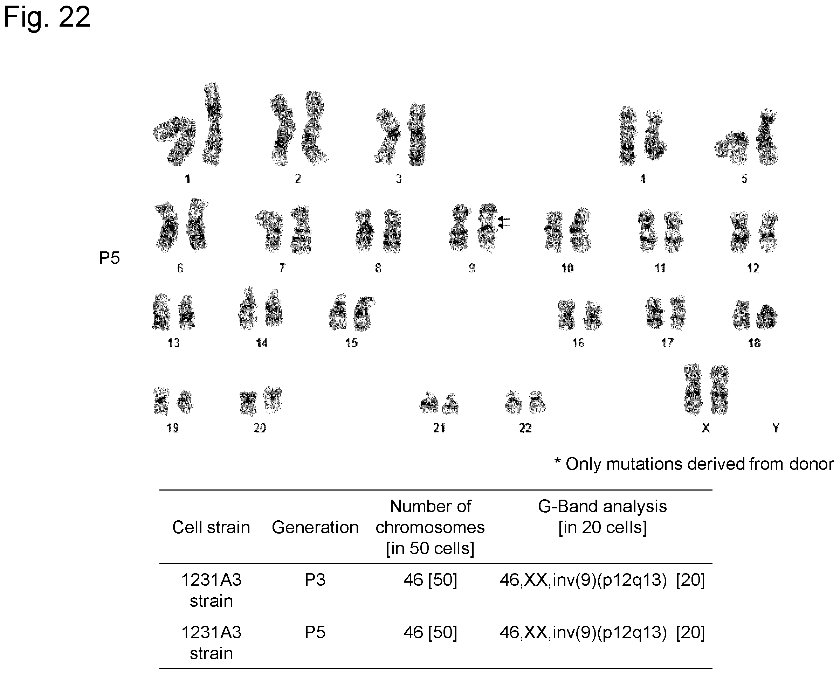

Figure 21 shows transition of results of quantitative real-time PCR analysis (see Example 4 for primers and analysis conditions used) for Example 13 and Reference Example 6. Each point with data missing in Figure 21 indicates a value below the detection limit. As shown in Figure 21, it is understood that, in spite of suspension culture, the relative gene expression levels of undifferentiation markers for Example 13 were kept at high levels comparable to those for the adherent culture in Reference Example 6 even in long-term culture. In addition, it is understood that the relative gene expression levels of representative markers for three germ layers for Example 13 were kept at low levels comparable to those for the adherent culture in Reference Example 6 even in long-term culture. Figure 22 shows results of G-Band analysis (outsourced to NIPPON GENE CO., LTD.) for the cells obtained in Example 13. As shown in Figure 22, it was found that no karyotypic abnormality newly occurred both in the third generation and in the fifth generation even through the suspension culture in Example 13.

From the above results shown in Figures 21 and 22, it was found that use of medium containing a TNKS inhibitor and a PKCβ inhibitor allows pluripotent stem cells to maintain the undifferentiation without the occurrence of karyotypic abnormality, even under stirring culture conditions involving applying high shear stress to pluripotent stem cells.

### <Example 14: Evaluation of Differentiation Capacity of iPS Cells After Long-Term Suspension Culture>

Cells in Example 13, which had been cryopreserved, were thawed, and suspended in in StemFit (R) AK02N medium containing Y-27632 with a final concentration of 10 µM, and some of the cells were stained with trypan blue and the number of viable cells was counted. StemFit (R) AK02N medium containing Y-27632 IWR-1-endo, and LY-333531 with final concentrations of 10 µM, 20 µM, and 1 µM, respectively, were applied to the cell suspension to prepare a cell suspension containing 2 × 10⁵ cells per 1 mL. Into a 6-well plate for suspension culture, 4 mL of the cell suspension was seeded per well. On a rotary shaker, the plate into which the cells had been seeded was rotated along a circle having a rotation width (diameter) of 25 mm in the horizontal plane at a speed of 90 rpm, and suspension culture was performed under an environment at 37°C and 5% CO₂ for 4 days.

Medium exchange was performed every day. Each medium exchange was performed in such a manner that the whole volume of medium containing a cell aggregate was first collected into a centrifuge tube, left to stand for about 5 minutes to precipitate the cell aggregate, the culture supernatant was then removed, and the resultant was gently resuspended in medium, which was returned into the original well. It should be noted that, in medium exchange, the final concentration of Y-27632 in medium was reduced to 6.9 µM 1 day after the initiation of culture, the final concentration of Y-27632 in medium was reduced to 3.71 µM after day 2 of culture, and the final concentration of Y-27632 in medium was reduced to 2.53 µM after day 3 of culture.

Subsequently, induction of differentiation into three germ layers was performed with the medium and additives in the medium changed as shown in Table 15.

**[Table 15]**

| Day -2 | | Day -1 | | Day 0 | | Day 1 | | Day 2 | | Day 3 | | Day 4 | | Day 5 | | Day 6 | | Day 7 | |
|---|---|---|---|---|---|---|---|---|---|---|---|---|---|---|---|---|---|---|---|
| Endoderm | StemFit 1x PSA 10 µM Y-27632 seeding density:2e5 cells/mL | | StemFit 1x PSA 5 µM Y-27632 | | [Endodermal differentiation medium (1)] | | [Endodermal differentiation medium (1)] | | [Endodermal differentiation medium (2)] | | [Endodermal differentiation medium (3)] | | endoderm | | | | | | |
| | | | | | RPMI1640 | | RPMI1640 | | RPMI1640 | | RPMI1640 | | | | | | | | |
| | | | | | 0.25% BSA | | 0.25% BSA | | 0.25% BSA | | 0.25% BSA | | | | | | | | |
| | | | | | 1x PSA | | 1x PSA | | 1x PSA | | 1x PSA | | | | | | | | |
| | | | | | 1 mM Sodium pyruvate | | 1 mM Sodium pyruvate | | 1 mM Sodium pyruvate | | 1 mM Sodium pyruvate | | | | | | | | |
| | | | | | 1x NEAA | | 1x NEAA | | 1x NEAA | | 1x NEAA | | | | | | | | |
| | | | | | 80 ng/mL | | 80 ng/mL | | 80 ng/mL | | 80 ng/mL | | | | | | | | |
| | | | | | ActivinA | | ActivinA | | ActivinA | | ActivinA | | | | | | | | |
| | | | | | 55 µM 2-mercaptethanol | | 55 µM 2-mercaptethanol | | 55 µM 2-mercaptethanol | | 55 µM 2-mercaptethanol | | | | | | | | |
| | | | | | 50 ng/mL FGF-2 | | 50 ng/mL FGF-2 | | | | 0.5% KSR | | | | | | | | |
| | | | | | 20 ng/mL BMP-4 | | 20 ng/mL BMP-4 | | | | | | | | | | | | |
| | | | | | 3 µM CHIR | | 3 µM CHIR | | | | | | | | | | | | |
| Mesoderm | | | | | [Mesodermal differentiation medium (1)] RPMI1640 B27 -Ins 7.5 µM CHIR | | [Mesodermal differentiation medium (2)] RPMI1640 B27 -Ins | | | | mesoderm | | | | | | | | |
| Ectoderm | | | | | StemFit w/o C solution | | StemFit w/o C solution | | StemFit w/o C_ solution | | StemFit w/o C_ solution | | StemFit w/o C solution | | StemFit w/o C_ solution | | StemFit w/o C_ solution | | ectoderm |
| | | | | | 10 µM | | 10 µM | | 10 µM | | 10 µM | | 10 µM | | | | | | |
| | | | | | SB431542 | | SB431542 | | SB431542 | | SB431542 | | SB431542 | | 10 µM | | 10 µM | | |
| | | | | | 10 µM DMH1 | | 10 µM DMH1 | | 10 µM DMH1 | | 10 µM DMH1 | | 10 µM | | SB431542 | | SB431542 | | |
| | | | | | | | | | | | | | DMH1 | | 10 µM | | 10 µM | | |
| | | | | | | | | | | | | | | | DMH1 | | DMH1 | | |

In the present Example, quantitative real-time PCR analysis was performed for CDX2 as a mesoderm marker, PDGFRa as a cardiogenic mesoderm marker, SOX17 as an endoderm marker, and PAX6 as an ectoderm marker. Primers and analysis conditions used for the quantitative real-time PCR analysis are the same as those in Example 4. For the cardiogenic mesoderm marker PDGFRa, the following primer set was used.
PDGFRa (Forward): 5'-GCTGAGCCTAATCCTCTGCC-3' (SEQ. ID. No.15)
PDGFRa (Reverse): 5'-ACTGCTCACTTCCAAGACCG-3' (SEQ. ID. No.16)

### <Reference Example 7: Evaluation of Differentiation Capacity of iPS Cells Subjected to Adherent Culture>

Cells in Reference Example 6, which had been cryopreserved, were thawed, and subjected to adherent culture in the same manner as in Reference Example 2, except that a culture dish having an inner diameter of 6 cm was used, the volume of medium was 4.2 mL, the seeding density was 1.5 × 10⁴ cells/cm², and the duration of culture was 4 days. After adherent culture, the human iPS cell 1231A3 strain was treated with Accutase (Innovative Cell Technologies, Inc.) for 5 minutes to detach the cells from the culture surface, and dispersed into single cells by pipetting. Thereafter, induction of differentiation into three germ layers was performed in the same manner as in Example 14.

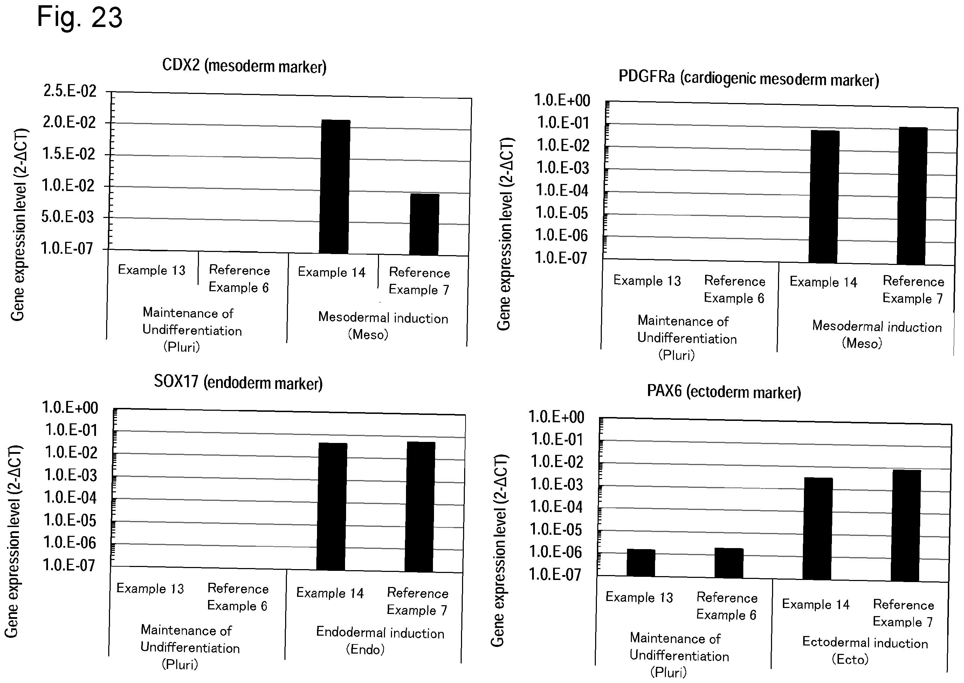

Figure 23 shows results of measurement of relative gene expression levels of the markers for three germ layers by quantitative real-time PCR analysis. As shown in Figure 23, it was revealed that the iPS cells subjected to long-term suspension culture in Example 14 had capability of inducing differentiation into three germ layers.

### <Regarding Examples 12 to 14>

As demonstrated above, it was revealed that use of medium containing a TNKS inhibitor and a PKCβ inhibitor allows pluripotent stem cells to be cultured for a long period of time through suspension culture, which is expected as a mass preparation method for pluripotent stem cells, with the proliferative ability, undifferentiation, and capability of inducing differentiation maintained. Pluripotent stem cells used in Examples 12 to 14 include those provided by using a production method equivalent to that for pluripotent stem cells for clinical use, such as those established in a feeder-free manner. Therefore, mass preparation of pluripotent stem cells for clinical use with the proliferative ability, undifferentiation, and capability of inducing differentiation maintained is also expected to be possible.

## Claims

1. A method for producing a pluripotent stem cell population, the method comprising the step of performing suspension culture of a pluripotent stem cell in a liquid medium comprising a PKCβ inhibitor and a TNKS inhibitor.

2. The method according to claim 1, wherein the concentration of the PKCβ inhibitor comprised in the liquid medium is 25 nM or more and 15 µM or less.

3. The method according to claim 1, wherein the concentration of the TNKS inhibitor comprised in the liquid medium is 90 nM or more and 40 µM or less.

4. The method according to claim 1, wherein the ratio of the concentrations of the PKCβ inhibitor and the TNKS inhibitor comprised in the liquid medium is in the range of 167:1 or higher and 1:1600 or lower.

5. The method according to claim 1, wherein the liquid medium comprises at least one selected from the group consisting of L-ascorbic acid, insulin, transferrin, selenium, and sodium hydrogen carbonate.

6. The method according to claim 1, wherein the liquid medium comprises FGF2 and/or TGF-β1.

7. The method according to claim 1, wherein the liquid medium comprises a ROCK inhibitor.

8. The method according to claim 7, wherein the ROCK inhibitor is Y-27632.

9. The method according to claim 1, wherein the step of performing suspension culture comprises the step of forming a cell aggregate.

10. The method according to claim 1, wherein the step of performing suspension culture comprises the step of collecting a cell aggregate.

11. The method according to claim 1, wherein, in the pluripotent stem cell population, the proportion of cells positive for OCT4 is 90% or higher, the proportion of cells positive for SOX2 is 90% or higher, and the proportion of cells positive for Nanog is 90% or higher.

12. The method according to claim 1, wherein the pluripotent stem cell is an ES cell and/or an induced pluripotent stem cell.

13. The method according to claim 1, wherein the suspension culture is performed until lactic acid concentration in the liquid medium reaches 5 to 15 mM.

14. The method according to claim 1, wherein the suspension culture is stirring culture with a blade tip speed of 0.05 m/s or higher and 1.37 m/s or lower.

15. A pluripotent stem cell population produced by the method according to any one of claims 1 to 14.

16. A differentiation inhibitor for pluripotent stem cells, the differentiation inhibitor comprising a PKCβ inhibitor and a TNKS inhibitor.

17. The differentiation inhibitor for pluripotent stem cells according to claim 16, wherein the concentration of the PKCβ inhibitor comprised therein is 50 nM or more and 200 mM or less.

18. The differentiation inhibitor for pluripotent stem cells according to claim 16, wherein the concentration of the TNKS inhibitor comprised therein is 180 nM or more and 113 mM or less.

19. The differentiation inhibitor for pluripotent stem cells according to claim 16, wherein the ratio of the concentrations of the PKCβ inhibitor and the TNKS inhibitor comprised in the differentiation inhibitor is in the range of 167:1 or higher and 1:1600 or lower.

20. A differentiation suppression kit for pluripotent stem cells, the differentiation suppression kit comprising the differentiation inhibitor for pluripotent stem cells according to any one of claims 16 to 19.
